# EUROPEAN PATENT SPECIFICATION

(11) **EP 2 218 708 B1**
(45) Date of publication and mention of the grant of the patent: **25.06.2014**
(21) Application number: 08856167.5
(22) Date of filing: 02.12.2008
(51) Int. Cl.: C07C 68/00, C07C 69/96, C07D 317/36, C07D 317/38, C07D 319/06, C07B 61/00

(54) **METHOD FOR PRODUCING CARBONATE COMPOUND**
VERFAHREN ZUR HERSTELLUNG EINER CARBONATVERBINDUNG
PROCÉDÉ DE PRODUCTION D'UN COMPOSÉ CARBONATE

(30) Priority: 03.12.2007 JP 2007312655; 13.12.2007 JP 2007321773; 13.08.2008 JP 2008208727
(43) Date of publication of application: 18.08.2010
(73) Proprietor: Asahi Glass Company, Limited, Chiyoda-ku Tokyo 100-8405 (JP)
(72) Inventor: OKAMOTO, Hidekazu, Tokyo 100-8405 (JP); TAJIMA, Kouhei, Tokyo 100-8405 (JP); OKAZOE, Takashi, Tokyo 100-8405 (JP)
(74) Representative: Müller-Boré & Partner Patentanwälte PartG mbB
(86) International application number: PCT/JP2008/071904
(87) International publication number: WO 2009/072502

(56) References cited:
- EP-A1- 0 557 167
- JP-A- 11 195 429
- JP-A- 2000 319 230
- JP-A- 2000 319 230
- JP-A- 2005 060 261
- JP-A- 2005 060 261
- RU-C1- 2 309 935
- US-A- 4 353 831
- US-A- 4 353 831

## Description

### TECHNICAL FIELD

The present invention relates to a novel process for producing a fluorine-containing carbonate compound.

### BACKGROUND ART

As processes for producing a carbonate compound, the following processes are known (JP 2005-60261, JP 2000-319230).
(1) A process for producing a cyclic carbonate by reacting carbon dioxide gas with an alkene oxide in the presence of a catalyst (see, e.g., Patent Document 1).
(2) A process for producing a dialkyl carbonate or a cyclic carbonate by reacting phosgene (COCl₂) with an alcohol (see, e.g., Patent Document 2).
(3) A process for producing a carbonate compound by an ester exchange reaction of a cyclic carbonate or dimethyl carbonate with an alcohol in the presence of an ester exchange reaction catalyst (see, e.g., Non-Patent Document 1).
(4) A process for producing a carbonate compound by reacting methyl chloroformate with an alcohol (see, e.g., Patent Document 2).

However, the process (1) involves a problem that only cyclic carbonates are produced and various carbonates cannot be selectively produced.

The process (2) involves problems that production facilities are corroded with hydrogen chloride produced as a by-product; phosgene has toxicity; and the like.

Since the process (3) is an equilibrium reaction, it involves problems that a large excess of an alcohol should be used for improving the yield of the objective product; it is difficult to separate and remove an asymmetrical carbonate compound produced as a by-product; and the like.

The process (4) involves problems that production facilities are corroded with hydrogen chloride produced as a by-product; and the like.

Also, as examples of reacting hexachloroacetone with an alcohol, the following examples have been reported.
(5) An example of synthesis of trichloroacetate by the reaction of hexachloroacetone with methanol (Non-Patent Document 2).
(6) An example wherein the formation of di(2-methyl-2-propen-1-yl) carbonate is confirmed by the reaction of hexachloroacetone with 2-methyl-2-propen-1-ol at room temperature or a lower temperature (Non-Patent Document 3).
(7) An example wherein a cyclic alkylene carbonate and chloroform are formed by the reaction of a vicinal diol compound (propylene glycol or the like) with hexachloroacetone in the presence of a base catalyst (a salt of a strong base with a weak acid) (Patent Document 3).
(8) An example wherein a cyclic alkylene carbonate and chloroform are formed by the reaction of a vicinal diol compound (propylene glycol or the like) with hexachloroacetone using a Group 2 or 3 metal hydrosilicate catalyst (Patent Document 4).

However, there has been unknown any example of synthesis of a fluorine-containing carbonate compound by the reaction of hexachloroacetone with a fluorine-containing alcohol. Many fluorine-containing alcohols are compounds whose OH groups exhibit a high acid dissociation degree owing to an electron-withdrawing property of a fluorine atom. In particular, the effect is remarkable in a compound having a fluorine atom at the β-position of the OH group. Therefore, it is anticipated that a fluorine-containing alcohol shows an extremely low reactivity to hexachloroacetone as compared with an alcohol having no fluorine atom.

Patent Document 1: JP-A-07-206847
Patent Document 2: JP-A-60-197639
Patent Document 3: U. S. Patent No. 4353831
Patent Document 4: Russian Patent No. 2309935
Non-Patent Document 1: Journal of Catalysis, 2006, Vol. 241, No. 1, p. 34-44
Non-Patent Document 2: Analytical Chemistry, 1983, Vol. 55, No. 8, p. 1222-1225
Non-Patent Document 3: Journal of Organic Chemistry, 1979, Vol. 44, No. 3, p. 359-363

### DISCLOSURE OF THE INVENTION

### PROBLEMS TO BE SOLVED BY THE INVENTION

The invention provides a novel production process capable of selectively producing various kinds of fluorine-containing carbonate compounds without any inhibition in high yields without using any toxic compounds such as phosgene and without producing any corrosive gases such as hydrogen chloride.

### MEANS FOR SOLVING THE PROBLEMS

The process for producing a carbonate compound of the invention is a process for producing a carbonate compound comprising reacting a compound represented by the following formula (1) with a fluorine-containing compound having at least one OH group to obtain a fluorine-containing compound having a carbonate bond: 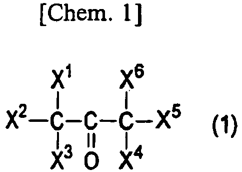 wherein X¹ to X³ each represents a hydrogen atom or a halogen atom, at least one of X¹ to X³ is a halogen atom, X⁴ to X⁶ each represents a hydrogen atom or a halogen atom, and at least one of X⁴ to X⁶ is a halogen atom.

The above fluorine-containing compound having at least one OH group is preferably a polyfluoroalkanemonool having 2 to 10 carbon atoms which has no fluorine atom at the α-position and may have an etheric oxygen atom, or a polyfluoroalkanediol having 3 to 10 carbon atoms which has no fluorine atom at the α-position and may have an etheric oxygen atom.

In the process for producing a carbonate compound of the invention, it is preferred that the reaction is carried out in the presence of a catalyst.

The catalyst preferably comprises a halogen salt. In the present specification, the halogen salt means a salt of a metallic or organic cation with a halogen ion.

The halogen salt preferably comprises one or more member selected from the group consisting of halogen salts of alkali metals, halogen salts of alkali earth metals, halogen salts of ammoniums, halogen salts of quaternary ammoniums, and ion-exchange resins having a halogen salt structure.

The halogen salt is preferably a fluoride of an alkali metal.

In the process for producing a carbonate compound of the invention, it is preferred that the reaction is carried out in the presence of the catalyst and a promoter, wherein the promoter is a solid acid catalyst.

The solid acid catalyst preferably comprises at least one member selected from the group consisting of metal oxides having a strong acid point, heteropoly acids, and cation-exchange resins.

The metal oxides having a strong acid point preferably comprise at lest one member selected from the group consisting of cerium oxide (CeO₂/Ce₂O₃), silica-alumina (SiO₂·Al₂O₃), γ-alumina (Al₂O₃), silica-magnesia (SiO₂-MgO), zirconia (ZrO₂), silica-zirconia (SiO₂·ZrO₂), ZnO·ZrO₂, and Al₂O₃·B₂O₃.

The fluorine-containing compound having a carbonate bond is preferably a compound represented by the following formula (31) or a compound represented by the following formula (32). 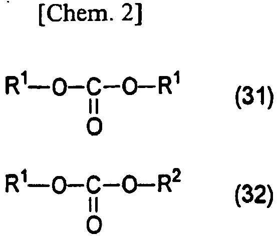 wherein R¹ and R² each represents a monovalent fluorine-containing aliphatic hydrocarbon group or a monovalent fluorine-containing aromatic hydrocarbon group, provided that R¹ and R² are not the same group.

The fluorine-containing compound having a carbonate bond is preferably a cyclic carbonate compound represented by the following formula (3a) or a linear carbonate compound represented by the following formula (3b). 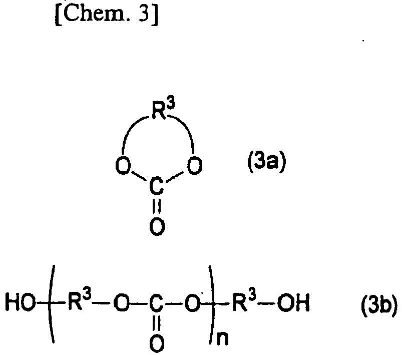 wherein R³ represents a divalent fluorine-containing aliphatic hydrocarbon group or a divalent fluorine-containing aromatic hydrocarbon group.

The fluorine-containing compound having at least one OH group preferably comprises at least one member selected from the group consisting of 2,2,2-trifluoroethanol, 2,2,3,3,3-pentafluoropropanol, 2,2,3,3-tetrafluoropropanol, 1-trifluoromethyl-2,2,2-trifluoro-1-ethanol (hexafluoroisopropanol), 2,2,3,4,4,4-hexafluorobutanol, 2,2,3,3,4,4,5,5-octafluoropentanol, 2,2-difluoro-2-(1,1,2,2-tetrafluoro-2-(pentafluoroethoxy)ethoxy)ethanol (CF₃CF₂OCF₂CF₂OCF₂CH₂OH), 2,2-difluoro-2-(tetrafluoro-2-(tetrafluoro-2-(pentafluoroethoxy)ethoxy)ethoxy)ethanol (CF₃CF₂OCF₂CF₂OCF₂CF₂OCF₂CH₂OH), 2,3,3,3-tetrafluoro-2-(1,1,2,3,3,3-hexafluoro-2-(1,1,2,2,3,3,3-heptafluoropropoxy)propoxy)-1-propanol (CF₃CF₂CF₂OCF(CF₃)CF₂OCF(CF₃)CH₂OH), 2,2,3,3-tetrafluoro-2-(1,1,2,2,3,3,3-heptafluoropropoxy)-1-propanol (CF₃CF₂CF₂OCF(CF₃)CH₂OH), and fluorine-containing phenols.

The fluorine-containing compound having at least one OH group preferably comprises at least one member selected from the group consisting of 3,3,3-trifluoro-1,2-propanediol, 4,4,4,3,3-pentafluoro-1,2-butanediol, 1,1,1,4,4,4-hexafluoro-2,3-butanediol, 3,3,4,4-tetrafluoro-1,6-hexanediol, 3,3,4,4,5,5,6,6-octafluoro-1,8-octanediol, tetrafluorohydroquinone, tetrafluororesorcinol, 2,2-bis(4-hydroxyphenyl)-hexafluoropropane and a compound represented by the following formula (X):

HO-CH₂CF₂-(CF₂CF₂O)ₘ-CF₂CH₂-OH (X)

wherein m is an integer of 2 to 30 and m is preferably 4 to 10.

In the process for producing a carbonate compound of the invention, it is preferred that the reaction is carried out with removing formed CHX¹X²X³ and/or CHX⁴X⁵X⁶ from the reaction system by distillation.

### ADVANTAGEOUS EFFECTS OF THE INVENTION

According to the process for producing a carbonate compound of the invention, various kinds of fluorine-containing carbonate compounds can be selectively produced without any inhibition in high yields without using any toxic compounds such as phosgene and without producing any corrosive gases such as hydrogen chloride. Moreover, in addition to cyclic fluorine-containing carbonates, oligomers or polymers of fluorine-containing carbonates having a reactive functional group at the terminal can be easily produced.

### BEST MODE FOR CARRYING OUT THE INVENTION

In the present specification, the compound represented by the formula (1) is referred to as compound (1). The compounds represented by the other formulae are also similarly referred to.

Moreover, in the specification, the fluorine-containing compound means a compound having a fluorine atom.

### <Carbonate compounds>

The carbonate compounds obtained by the production process of the invention are compounds having a carbonate bond (-O-C(=O)-O-).

Examples of the carbonate compound include the compound (31), the compound (32), the compound (3a), the compound (3b), and the branched carbonate compound having two or more terminal OH groups (hereinafter referred to as branched carbonate compound).

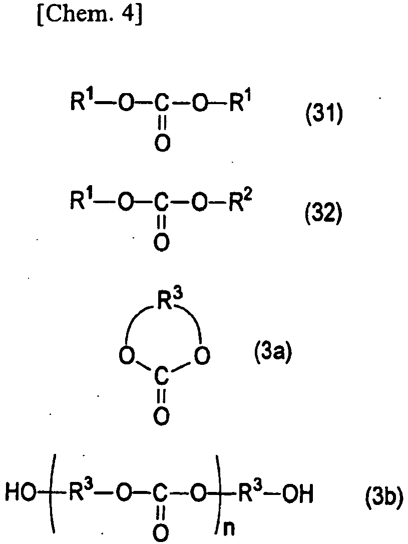

### (Compound (31))

R¹ represents a monovalent fluorine-containing aliphatic hydrocarbon group or a monovalent fluorine-containing aromatic hydrocarbon group. R¹'s on the left and right sides are the same.

The monovalent fluorine-containing aliphatic hydrocarbon group may contain an etheric oxygen atom.

The monovalent fluorine-containing aliphatic hydrocarbon group may be linear, branched, or cyclic.

R¹ may have a substituent. As the substituent, a halogen atom (excluding a fluorine atom) is preferred in view of usefulness of the compound (31).

As the monovalent fluorine-containing aliphatic hydrocarbon group, a polyfluoroalkyl group having 2 to 10 carbon atoms which has no fluorine atom at the α-position and may have an etheric oxygen atom is preferred in view of usefulness of the compound (31). As the alkyl group in the polyfluoroalkyl group having 2 to 10 carbon atoms, an ethyl group, an n-propyl group, an i-propyl group, a t-butyl group, or an n-pentyl group is preferred. As the alkyl group in the polyfluoroalkyl group having an etheric oxygen atom, an (ethoxy(ethoxy))ethyl group, an (etoxy(ethoxy(ethoxy)))ethyl group, a (propoxy)propyl group, or a (propoxy(propoxy))propyl group is preferred.

The monovalent fluorine-containing aromatic hydrocarbon group may have a substituent of an aliphatic hydrocarbon group or an aromatic hydrocarbon group on the aromatic nuclei.

Examples of the monovalent fluorine-containing aromatic hydrocarbon group include fluorine-containing phenyl groups, fluorine-containing methylphenyl groups, fluorine-containing ethylphenyl groups and fluorine-containing naphthyl groups, and a fluorine-containing phenyl group is preferred in view of usefulness of the compound (31).

### (Compound (32))

R¹ and R² each represents a monovalent fluorine-containing aliphatic hydrocarbon group or a monovalent fluorine-containing aromatic hydrocarbon group and R¹ and R² are not the same group.

The monovalent fluorine-containing aliphatic hydrocarbon group may contain an etheric oxygen atom.

The monovalent fluorine-contairiing aliphatic hydrocarbon group may be linear, branched, or cyclic.

R¹ and R² may have a substituent. As the substituent, a halogen atom (excluding a fluorine atom) is preferred in view of usefulness of the compound (32).

As the monovalent fluorine-containing aliphatic hydrocarbon group, a polyfluoroalkyl group having 2 to 10 carbon atoms which has no fluorine atom at the α-position and may have an etheric oxygen atom is preferred in view of usefulness of the compound (32). As the alkyl group in the polyfluoroalkyl group having 2 to 10 carbon atoms, an ethyl group, an n-propyl group, an i-propyl group, a t-butyl group, or an n-pentyl group is preferred. As the alkyl group in the polyfluoroalkyl group having an etheric oxygen atom, an (ethoxy(ethoxy))ethyl group, an (etoxy(ethoxy(ethoxy)))ethyl group, a (propoxy)propyl group, or a (propoxy(propoxy))propyl group is preferred.

The monovalent fluorine-containing aromatic hydrocarbon group may have a substituent of an aliphatic hydrocarbon group or an aromatic hydrocarbon group on the aromatic nucleus.

As the monovalent fluorine-containing aromatic hydrocarbon group, an aromatic hydrocarbon group having 6 to 16 carbon atoms is preferred.

Examples of the monovalent fluorine-containing aromatic hydrocarbon group include fluorine-containing phenyl groups, fluorine-containing methylphenyl groups, fluorine-containing ethylphenyl groups and fluorine-containing naphthyl groups, and a fluorine-containing phenyl group is preferred in view of usefulness of the compound (32).

The asymmetrical compound (32) is known to have a melting point lower that that of the symmetrical compound (31) and is predicted to be superior in the case where it is used as a solvent or the like.

### (Compound (3 a))

The compound (3a) is a cyclic carbonate compound.

R³ represents a divalent fluorine-containing aliphatic hydrocarbon group or a divalent fluorine-containing aromatic hydrocarbon group.

The divalent fluorine-containing aliphatic hydrocarbon group may contain an etheric oxygen atom.

The divalent fluorine-containing aliphatic hydrocarbon group may be linear, branched, or cyclic.

R³ may have a substituent. As the substituent, a halogen atom (excluding a fluorine atom) is preferred in view of usefulness of the compound (3a).

As R³, a polyfluoroalkylene group having 3 to 10 carbon atoms which has no fluorine atom at the α-position and may have an etheric oxygen atom is preferred in view of usefulness of the compound (3a). As the alkyl group in the polyfluoroalkylene group having 3 to 10 carbon atoms, -CH₂CH(CH₃)-, - CH₂CH(C₂H₅)-, or -CH₂CH₂CH₂- is preferred.

As the compound (3a), a compound obtained by replacing a part or all of the hydrogen atom(s) of the following compound by fluorine atom(s) is preferred: 1,2-propylene carbonate, 1,3-propylene carbonate, or 1,2-butylene carbonate.

### (Compound (3b))

The compound (3b) is an oligomer or polymer having an OH group, which is a reactive functional group, at the terminal.

R³ represents a divalent fluorine-containing aliphatic hydrocarbon group or a divalent fluorine-containing aromatic hydrocarbon group. In the case where a plurality of R³'s are present in the compound (3b), R³'s may be a single kind or may be two or more kinds.

The divalent fluorine-containing aliphatic hydrocarbon group may contain an etheric oxygen atom.

The divalent fluorine-containing aliphatic hydrocarbon group may be linear, branched, or cyclic.

R³ may have a substituent. As the substituent, a halogen atom (excluding a fluorine atom) is preferred in view of usefulness of the compound (3b).

As R³, a polyfluoroalkylene group having 3 to 64 carbon atoms which has no fluorine atom at the α-position and may have an etheric oxygen atom is preferred in view of usefulness of the compound (3b), a polyfluoroalkylene group having 3 to 14 carbon atoms is more preferred, and a polyfluoroalkylene group having 3 to 10 carbon atoms is most preferred. As the alkyl group in the polyfluoroalkylene group having 3 to 10 carbon atoms, -CH₂CH₂CH(CH₃)CH₂CH₂-, - CH₂CH₂CH₂CH₂CH₂CH₂-, -CH₂CH₂CH₂CH₂-, or -CH₂CH₂CH₂- is preferred. As the alkylene group in the polyfluoroalkylene group having an etheric oxygen atoms, a group represented by the following formula (XI) is preferred.

-CH₂CH₂O(CH₂CH₂O)ₘ-CH₂CH₂- (XI)

wherein m is an integer of 2 to 30 and m is preferably 4 to 10.

Moreover, as R³, a group obtained by replacing a part or all of the hydrogen atom(s) of a group represented by the following formula (4) by fluorine atom(s) is preferred.

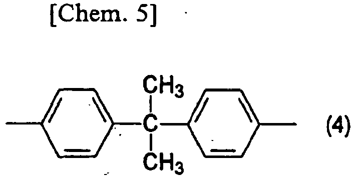

The symbol n in formula (3b) represents an integer of 1 to 1000, preferably an integer of 5 to 100, and more preferably an integer of 10 to 50. In this connection, the compound (3b) as a reaction product is usually obtained as a mixture of compounds having different n numbers.

As the compound (3b), a compound obtained by replacing a part or all of the hydrogen atom(s) of the following compound by fluorine atom(s) is preferred: poly(1,3-propylene carbonate), poly(1,4-butylene carbonate), poly(3-methyl-1,5-pentylene carbonate), poly(1,6-hexylene carbonate), poly(polyethylene oxide-α,ω-carbonate), or copolymers having these repeating units.

### (Branched carbonate compound)

Examples of the branched carbonate compound include branched oligomers and branched polymers, each having more than two terminal OH groups. The branched carbonate compound having more than two terminal OH groups includes those having three or more terminal OH groups and mixtures of those having two terminal OH groups and those having three or more terminal OH groups. In the case of the mixtures, the number of OH groups is judged with the average value, and "more than two" represents, for example, 2.05, 2.1, or the like.

### <Process for producing carbonate compound>

The process for producing a carbonate compound of the invention is a process for producing a carbonate compound by reacting the compound (1) with a fluorine-containing compound having one OH group or a fluorine-containing compound having two or more OH groups, optionally, in the presence of a catalyst.

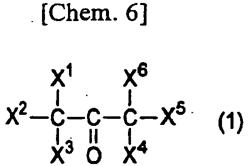

### (Compound (1))

X¹ to X⁶ is chlorine atoms. From the viewpoint that chloroform is obtained as a by-product, they are most preferably all chlorine atoms.

Compound (1) is hexachloroacetone, In view of capability of simultaneous production of industrially useful chloroform in a high yield, hexachloroacetone is preferred.

Among the compounds (1), chloroacetones can be easily produced by the processes of chlorinating acetone as described in JP-B-60-52741 and JP-B-61-16255. Moreover, partially fluorinated compounds can be easily produced by fluorinating chloroacetones with hydrogen fluoride as described in US Patent No. 6235950.

### (Catalyst)

In the process for producing a carbonate compound of the invention, it is preferred to obtain the fluorine-containing compound having a carbonate bond in the presence of a catalyst. By the use of the catalyst, the reaction can be efficiently carried out and the yields can be improved.

Examples of the catalyst include one or more members selected from the group consisting of alkali metals, alkaline earth metals; alkali metal hydrides, alkaline earth metal hydrides; alkali metal hydroxides, alkaline earth metal hydroxides; phase transfer catalysts; halogen salts of alkali metals; halogen salts of alkaline earth metals; halogen salts of ammoniums; ion-exchange resins; compounds of one or more metals selected from the group consisting of tin, titanium, aluminum, tungsten, molybdenum, zirconium, and zinc; and transesterification reaction catalysts.

Examples of the alkali metals include Li, Na, K, Rb and Cs.

Examples of the alkaline earth metals include Be, Ca and Sr.

Examples of the alkali metal hydrides include LiH, NaH, KH, RbH and CsH.

Examples of the alkaline earth metal hydrides include BeH₂, CaH₂ and SrH₂.

Examples of the alkali metal hydroxides include LiOH, NaOH, KOH, RbOH and CsOH.

Examples of the alkaline earth metal hydroxides include Be(OH)₂, Ca(OH)₂ and Sr(OH)₂.

Examples of the phase transfer catalysts include quaternary ammonium salts, quaternary phosphonium salts, quaternary arsonium salts, and sulfonium salts.

Examples of the quaternary ammonium salts include compounds (5): 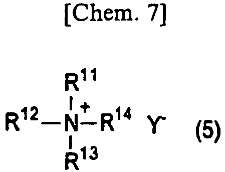 wherein R¹¹ to R¹⁴ each represents a hydrocarbon group and Y⁻ represents an anion.

Examples of R¹¹ to R¹⁴ include alkyl groups, cycloalkyl groups, alkenyl groups, cycloalkenyl groups, aryl groups, alkylaryl groups and aralkyl groups, and alkyl groups, aryl groups or aralkyl groups are preferred.

The total number of the carbon atoms of R¹¹ to R¹⁴ is preferably 4 to 100 per one molecule of R¹¹R¹²R¹³R¹⁴N⁺.

R¹¹ to R¹⁴ may be the same group or may be different groups.

R¹¹ to R¹⁴ may be substituted with functional group(s) inert under reaction conditions. Although the inert functional group varies depending on the reaction conditions, examples thereof include a halogen atom, an ester group, a nitrile group, an acyl group, a carboxyl group and an alkoxyl group.

R¹¹ to R¹⁴ may be combined with each other to form a heterocyclic ring including a nitrogen-containing heterocyclic ring or the like.

R¹¹ to R¹⁴ may be a part of a polymer compound.

Examples of R¹¹R¹²R¹³R¹⁴N⁺ include a tetramethylammonium ion, a tetraethylammonium ion, a tetra-n-propylammonium ion, a tetra-n-butylammonium ion, a tri-n-octylmethylammonium ion, a cetyltrimethylammonium ion, a benzyltrimethylammonium ion, a benzyltriethylammonium ion, a cetylbenzyldimetylammonium ion, a cetylpyridinium ion, an n-dodecylpyridinium ion, a phenyltrimetylammonium ion, a phenyltriethylammonium ion, an N-benzylpicolinium ion, a pentamethonium ion, and a hexamethonium ion.

Examples of Y⁻ include a chlorine ion, a fluorine ion, a bromine ion, an iodine ion, a sulfate ion, a nitrate ion, a phosphate ion, a perchlorate ion, a hydrogen sulfate ion, a hydroxide ion, an acetate ion, a benzoate ion, a benzenesulfonate ion, and a p-toluenesulfonate ion, and a chlorine ion, a bromine ion, an iodine ion, a hydrogen sulfate ion or a hydroxide ion is preferred.

As the compound (5), in view of versatility and reactivity of the compound (5), a combination of the following R¹¹R¹²R¹³R¹⁴N⁺ and the following Y⁻ is preferred.

R¹¹R¹²R¹³R¹⁴N⁺: a tetramethylammonium ion, a tetraethylammonium ion, a tetra-n-propylammonium ion, a tetra-n-butylammonium ion, or a tri-n-octylmethylammonium ion.

Y⁻: a fluorine ion, a chlorine ion, or a bromine ion.

Examples of the quaternary phosphonium salts include compounds (6): 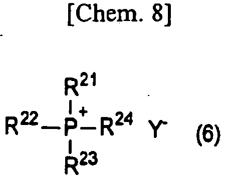 wherein R²¹ to R²⁴ each represents a hydrocarbon group and Y⁻ represents an anion.

Examples of R²¹ to R²⁴ include alkyl groups, cycloalkyl groups, alkenyl groups, cycloalkenyl groups, aryl groups, alkylaryl groups and aralkyl groups, and alkyl groups, aryl groups, or aralkyl groups are preferred.

The total number of the carbon atoms of R²¹ to R²⁴ is preferably 4 to 100 per one molecule of R²¹R²²R²³R²⁴P⁺.

R²¹ to R²⁴ may be the same group or may be different groups.

R²¹ to R²⁴ may be substituted with a functional group inert under reaction conditions. Although the inert functional group varies depending on the reaction conditions, and examples thereof include a halogen atom, an ester group, a nitrile group, an acyl group, a carboxyl group and an alkoxyl group.

Examples of R²¹R²²R²³R²⁴P⁺ include a tetraethylphosphonium ion, a tetra-n-butylphosphonium ion, a tri-n-octylethylphosphonium ion, a cetyltriethylphosphonium ion, a cetyltri-n-butylphosphonium ion, an n-butyltriphenylphosphonium ion, an n-amyltriphenylphosphonium ion, a methyltriphenylphosphonium ion, a benzyltriphenylphosphonium ion, and a tetraphenylphosphonium ion.

Examples of Y⁻ include a chlorine ion, a fluorine ion, a bromine ion, an iodine ion, a sulfate ion, a nitrate ion, a phosphate ion, a perchlorate ion, a hydrogen sulfate ion, a hydroxide ion, an acetate ion, a benzoate ion, a benzenesulfonate ion, and a p-toluenesulfonate ion, and a fluorine ion, a chlorine ion or a bromine ion is preferred.

Examples of the quaternary arsonium salts include compounds (7): 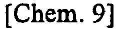 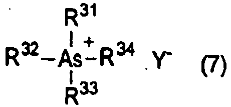 wherein R³¹ to R³⁴ each represents a hydrocarbon group and Y⁻ represents an anion.

Examples of R³¹ to R³⁴ include alkyl groups, cycloalkyl groups, alkenyl groups, cycloalkenyl groups, aryl groups, alkylaryl groups and aralkyl groups, and alkyl groups, aryl groups or aralkyl groups are preferred.

The total number of the carbon atoms of R³¹ to R³⁴ is preferably 4 to 100 per one molecule of R³¹R³²R³³R³⁴As⁺_{.}

R³¹ to R³⁴ may be the same group or may be different groups.

R³¹ to R³⁴ may be substituted with a functional group inert under reaction conditions. Although the inert functional group varies depending on the reaction conditions, examples thereof include a halogen atom, an ester group, a nitrile group, an acyl group, a carboxyl group and an alkoxyl group.

Examples of the compound (7) include triphenylmethylarsonium fluoride, tetraphenylarsonium fluoride, triphenylmethylarsonium chloride, tetraphenylarsonium chloride, tetraphenylarsonium bromide, and polymer derivatives thereof.

Examples of the sulfonium salts include compounds (8): 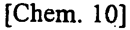 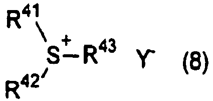 wherein R⁴¹ to R⁴³ each represents a hydrocarbon group and Y⁻ represents an anion.

Examples of R⁴¹ to R⁴³ include alkyl groups, cycloalkyl groups, alkenyl groups, cycloalkenyl groups, aryl groups, alkylaryl groups and aralkyl groups, and alkyl groups, aryl groups or aralkyl groups are preferred.

The total number of the carbon atoms of R⁴¹ to R⁴³ is preferably 4 to 100 per one molecule of R⁴¹R⁴²R⁴³S⁺_{.}

R⁴¹ to R⁴³, may be the same group or may be different groups.

R⁴¹ to R⁴³ may be substituted with a functional group inert under reaction conditions. Although the inert functional group varies depending on the reaction conditions, examples thereof include a halogen atom, an ester group, a nitrile group, an acyl group, a carboxyl group and an alkoxyl group.

R⁴¹ to R⁴³ may be combined with each other to form a heterocyclic ring including a nitrogen-containing heterocyclic ring.

R⁴¹ to R⁴³ may be a part of a polymer compound.

Examples of Y⁻ include various anions. A halogen ion is preferred and a fluorine ion, a chlorine ion, or a bromine ion is more preferred.

Examples of the compound (8) include di-n-butylmethylsulfonium iodide, a tri-n-butylsulfonium tetrafluoroborate, a dihexylmethylsulfonium iodide, dicyclohexylmethylsulfonium iodide, dodecylmethylethylsulfonium chloride, and tris(diethylamino)sulfonium difluorotrimethylsilicate.

Examples of the halogen salts of alkali metals include LiF, LiCl, LiBr, NaF, NaCl, NaBr, KF, KCl, KBr, RbF, RbCl, RbBr, CsF, CsCl and CsBr.

Examples of the halogen salts of alkali earth metals include BeF₂, BeCl₂, BeBr₂, CaF₂, CaCl₂, CaBr₂, SrF₂, SrCl₂ and SrBr₂.

Examples of the halogen salts of ammoniums include NH₄F, NH₄Cl and NH₄Br.

The ion-exchange resins include cation type ion-exchange resins and anion type ion-exchange resins. Examples of commercially available products include DIAION (registered trademark) series (manufactured by Mitsubishi Chemical Corporation), Amberlite (registered trademark) series (manufactured by Rohm and Haas Company) and Amberlyst (registered trademark) series (manufactured by Rohm and Haas Company).

As the ion-exchange resins, in view of the reaction rate, anion type ion-exchange resins where a halogen ion is used as an anion (ion-exchange resins having a halogen salt structure) are preferred.

Examples of the compounds of one or more metals selected from the group consisting of tin, titanium, aluminum, tungsten, molybdenum, zirconium, and zinc include titanium compounds (tetrabutyl titanates, tetrapropyl titanates, tetraethyl titanates, tetramethyl titanates, etc.), organotin compounds (tin octylate, monobutyltin oxide, monobutyltin tris(2-ethylhexanoate), dibutyltin oxide, dibutyltin laurate, dibutyltin diacetate, monobutyltin hydroxyoxide, etc.), stannous oxide, tin halides (stannous chloride, stannous bromide, stannous iodide, etc.), and aluminum chloride.

The transesterification reaction catalysts include alkali or acid catalysts (alcoholates of alkali metals, butyllithium, p-toluenesulfonic acid, sulfuric acid, perchloric acid, BF₃, etc.) and the like.

As the catalyst, in view of easy handling at industrial use, reactivity, and selectivity to objective products, a halogen salt is preferred.

The halogen salt is preferably one or more member selected from the group consisting of halogen salts of alkali metals, halogen salts of alkali earth metals, halogen salts of ammoniums, halogen salts of quaternary ammoniums, and ion-exchange resins having a halogen salt structure.

As the halogen salt, in view of reactivity and utilization in an industrial scale, a fluoride of an alkali metal (NaF, KF, or the like) or a quaternary ammonium bromide is preferred.

The halogen salt may be supported on a metal oxide or a composite oxide. Examples of the compound include soda lime.

### (Promoter)

In the process for producing a carbonate compound of the invention, it is preferred to obtain the fluorine-containing compound having a carbonate bond in the presence of a catalyst and a promoter. Using the promoter, catalyst activity can be improved.

As the promoter, a solid acid catalyst is used.

The solid acid catalyst is preferably at least one member selected from the group consisting of metal oxides having a strong acid point, heteropoly acids, and cation-exchange resins.

Examples of the metal oxides having a strong acid point include SiO₂·Al₂O₃, SiO₂·MgO, SiO₂·ZrO₂, Al₂O₃·B₂O₃, Al₂O₃, ZrO₂, ZnO·ZrO₂, CeO₂, Ce₂O₃, and various zeolites. In view of acid strength and reaction selectivity, at least one member selected from the group consisting of cerium oxide (CeO₂/Ce₂O₃), silica-alumina (SiO₂·Al₂O₃), γ-alumina (Al₂O₃), silica-magnesia (SiO₂·MgO), zirconia (ZrO₂), silica-zirconia (SiO₂·ZrO₂), ZnO·ZrO₂, and Al₂O₃·B₂O₃ is preferred.

### (Process for producing compound (31))

The compound (31) is produced by reacting the compound (1) with a compound (21), optionally, in the presence of a catalyst.

[Chem. 11]

R¹-OH (21)

Examples of the compound (21) include a monovalent fluorine-containing aliphatic alcohol and a monovalent fluorine-containing phenol.

As the monovalent fluorine-containing aliphatic alcohol, in view of versatility on industrial use, a fluorine-containing saturated aliphatic alcohol is preferred and a polyfluoroalkanemonool having 2 to 10 carbon atoms which has no fluorine atom at the α-position and may have an etheric oxygen atom is more preferred.

Examples of the polyfluoroalkanemonool having 2 to 10 carbon atoms include 2,2,2-trifluoroethanol, 2,2,3,3,3-pentafluoropropanol, 2,2,3,3-tetrafluoropropanol, 1-trifluoromethyl-2,2,2-trifluoro-1-ethanol (hexafluoroisopropanol), 3,3,3-trifluoropropanol, 3-fluoropropanol, 2-fluoropropanol, 2-methyl-2-fluoroethanol, 2,2,3,4,4,4-hexafluorobutanol, 2,2,3,3,4,4,5,5-octafluoropentanol, 3,3,4,4,4-pentafluorobutanol, 4,4,5,5,5-pentafluoropentanol, 3,3,4,4,5,5,6,6,6-nonafluorohexanol, 2,2-difluoro-2-(1,1,2,2-tetrafluoro-2-(pentafluoroethoxy)ethoxy)ethanol (CF₃CF₂OCF₂CF₂OCF₂CH₂OH), 2,2-difluoro-2-(tetrafluoro-2-(tetrafluoro-2-(pentafluoroethoxy)ethoxy)ethoxy)ethanol (CF₃CF₂OCF₂CF₂OCF₂CF₂OCF₂CH₂OH), 2,3,3,3-tetrafluoro-2-(1,1,2,3,3,3-hexafluoro-2-(1,1,2,2,3,3,3-heptafluoropropoxy)propoxy)-1-propanol (CF₃CF₂CF₂OCF(CF₃)CF₂OCF(CF₃)CH₂OH), and 2,2,3,3-tetrafluoro-2-(1,1,2,2,3,3,3-heptafluoropropoxy)-1-propanol (CF₃CF₂CF₂OCF(CF₃)CH₂OH).

As the monovalent fluorine-containing aliphatic alcohol, in view of usefulness of the compound (31), a polyfluoroalkanemonool having 2 to 6 carbon atoms which has no fluorine atom at the α-position and may have an etheric oxygen atom is more preferred. Specifically, 2,2,2-trifluoroethanol, 2,2,3,3,3-pentafluoropropanol, 2,2,3,3-tetrafluoropropanol, 1-trifluoromethyl-2,2,2-trifluoro-1-ethanol (hexafluoroisopropanol), 3,3,3-trifluoropropanol, 3-fluoropropanol, 2-fluoropropanol, 2-methyl-2-fluoroethanol, 2,2,3,4,4,4-hexafluorobutanol, 2,2,3,3,4,4,5,5-octafluoropentanol, 2,2-difluoro-2-(1,1,2,2-tetrafluoro-2-(pentafluoroethoxy)ethoxy)ethanol (CF₃CF₂OCF₂CF₂OCF₂CH₂OH), or 2,2,3,3-tetrafluoro-2-(1,1,2,2,3,3,3-heptafluoropropoxy)-1-propanol (CF₃CF₂CF₂OCF(CF₃) CH₂OH) is more preferred, and 2,2,2-trifluoroethanol, 2,2,3,3,3-pentafluoropropanol, 2,2,3,3-tetrafluoropropanol, 1-trifluoromethyl-2,2,2-trifluoro-1-ethanol (hexafluoroisopropanol), 2,2,3,4,4,4-hexafluorobutanol, or 2,2,3,3,4,4,5,5-octafluoropentanol is most preferred.

As the monovalent fluorine-containing phenol, in view of usefulness of the compound (31), a fluorine-containing phenol is preferred.

The ratio of the first charged molar amount of the compound (21) to the first charged molar amount of the compound (1) (compound (21)/compound (1)) is preferably more than 1, more preferably 1.5 or more, and particularly preferably 2 or more in view of improving the yield of the compound (31). By regulating the ratio to more than 1, the reaction equilibrium shifts to the compound (31) side, thereby the reaction yield being improved.

The amount of the catalyst in the case where the catalyst is used in the reaction is variously selected depending on the catalyst, but is preferably 0.01 to 30% by mass and, in consideration of reactivity and a catalyst removal step after the reaction, is more preferably 0.1 to 10% by mass based on the substrate.

The amount of the promoter in the case where the promoter is used in the reaction is variously selected depending on the promoter, but is preferably 0.01 to 30% by mass and, in consideration of reactivity and a promoter removal step after the reaction, is more preferably 0.1 to 10% by mass based on the substrate.

Since the compound (21) mostly has a low compatibility with the compound (1), the reaction sometimes forms a heterogeneous system at an early reaction stage. Accordingly, in the reaction, a solvent may be used for the purpose of promoting the reaction. However, when volume efficiency of a reactor and loss of the objective product at a solvent separation step are considered, it is preferred to carry out the reaction without any solvent, if possible.

The solvent may be one stably present at the reaction temperature and showing a high solubility of the starting materials and, in view of capability of separation of the compound (1), the compound (21), the compound (31), and by-products by distillation after the reaction, it is preferred to use a solvent having a boiling point different from that of each of these compounds or to use the compound (31) as the solvent.

As the solvent, carbonate compounds different in boiling point, the compound (31), ethers having a relatively high boiling point are preferred. Specific examples thereof include ethylene carbonate, propylene carbonate, dimethyl carbonate, diethyl carbonate, dipropyl carbonate, dibutyl carbonate, dioctyl carbonate, glyme, diglyme, triglyme and tetraglyme.

When the effect of using the solvent is considered, the amount of the solvent is preferably an amount so that the concentration of the substrate becomes 10 to 80% by mass. However, in the case of a substrate where the effect of using the solvent is not so much observed, no solvent (substrate concentration of 100% by mass) is preferred in view of separation.

In the invention, at least a part of the reaction between the compound (1) and the compound (21) is preferably carried out at a reaction temperature of 40 to 200°C.

When the reaction temperature is lower than 40°C, the yield of the carbonate compound is extremely low. When the reaction temperature exceeds 200°C, decrease in yield owing to the decomposition of the compound (1) to be used as a starting material becomes remarkable. When the reaction temperature falls within the above range, the carbonate compound can be produced in high yields at a reaction rate capable of industrial use.

The reaction temperature is more preferably 40 to 160°C, more preferably 50 to 150°C, and particularly preferably 60 to 140°C.

The efficiency of the reaction can be improved by carrying out the reaction at different reaction temperatures at the early reaction stage and at the later reaction stage. This is because the substitution reactions of the two functional groups in the compound (1) proceeds stepwise and the reaction rate of the first step substitution reaction is high but the reaction rate of the second substitution reaction is comparatively low. Since the first step substitution reaction easily proceeds at a relatively low temperature of about 0 to 100°C and is a reaction with severe heat generation for a while, the reaction is preferably allowed to proceed at a relatively low temperature at the early reaction stage. The second step substitution reaction is carried out at a relatively high temperature of about 50 to 200°C in view of the reaction rate.

The reaction pressure is usually atmospheric pressure. Depending on the vapor pressure of the compound (21) at the reaction temperature, it is preferred to apply pressure.

In the present reaction, CHX¹X²X³ and/or CHX⁴X⁵X⁶ (chloroform and the like), which are halogenated methanes having a low boiling point, are formed as the reaction proceeds. Accordingly, in order to improve the reaction yield by shifting the reaction equilibrium to the compound (31) side and to complete the reaction stoichiometrically, it is preferred to carry out the reaction with removing the formed CHX¹X²X³ and/or CHX⁴X⁵X⁶ from the reaction system by distillation.

As a method for removing halogenated methanes by distillation, a reaction distillation mode utilizing the fact that the halogenated methanes each has a low boiling point as compared with the compound (21) and the compound (31) is preferred from the viewpoint of easy implementation.

### (Process for producing compound (32))

The compound (32) is preferably produced by reacting the compound (1) with the compound (21) optionally in the presence of a catalyst to obtain a compound (11a) and/or a compound (11b) (hereinafter the compound (11a) and the compound (11b) are collectively referred to as compound (11)) and successively reacting the compound (11) with a compound (22).

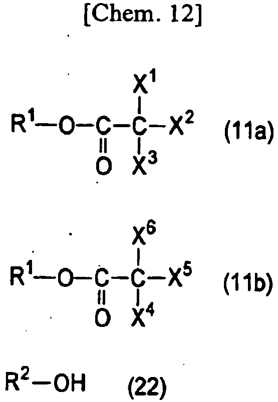

Moreover, the compound (1), the compound (21), and the compound (22) may be reacted at the same time. In that case, the compound (32), the compound (31), and the compound (33) are obtained as a mixture.

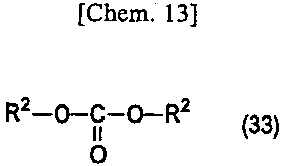

Examples of the compound (22) include the above-mentioned monovalent fluorine-containing aliphatic alcohols and monovalent fluorine-containing phenols. However, as the compound (22), an alcohol different from the compound (21) is used.

As the monovalent fluorine-containing aliphatic alcohol, in view of usefulness of the compound (32), a fluoroalkanemonool having 2 to 6 carbon atoms which has no fluorine atom at the α-position and may have an etheric oxygen atom is preferred, and a fluoroalkanemonool having 2 to 4 carbon atoms which has no fluorine atom at the α-position and may have an etheric oxygen atom is more preferred.

As the fluoroalkanemonool having 2 to 6 carbon atoms, 2,2,2-trifluoroethanol, 2,2,3,3,3-pentafluoropropanol, 2,2,3,3-tetrafluoropropanol, 1-trifluoromethyl-2,2,2-trifluoro-1-ethanol (hexafluoroisopropanol), 3,3,3-trifluoropropanol, 3-fluoropropanol, 2-fluoropropanol, 2-methyl-2-fluoroethanol, 2,2,3,4,4,4-hexafluorobutanol, 2,2,3,3,4,4,5,5-octafluoropentanol, 2,2-difluoro-2-(1,1,2,2-tetrafluoro-2-(pentafluoroethoxy)ethoxy)ethanol (CF₃CF₂OCF₂CF₂OCF₂CH₂OH), or 2,2,3,3-tetrafluoro-2-(1,1,2,2,3,3,3-heptafluoropropoxy)-1-propanol (CF₃CF₂CF₂OCF(CF₃)CH₂OH) is preferred.

As the fluoroalkanemonool having 2 to 4 carbon atoms, 2,2,2-trifluoroethanol, 2,2,3,3,3-pentafluoropropanol, 2,2,3,3-tetrafluoropropanol, 1-trifluoromethyl-2,2,2-trifluoro-1-ethanol (hexafluoroisopropanol), or 2,2,3,4,4,4-hexafluorobutanol is preferred.

As the monovalent fluorine-containing phenol, in view of usefulness of the compound (32), a fluorine-containing phenol is preferred.

The ratio of the first charged molar amounts of the compound (21) and the compound (22) to the first charged molar amount of the compound (1) ((compound (21)+ compound (22))/compound (1)) is preferably more than 1, more preferably 1.5 or more, and particularly preferably 2 or more.

Moreover, in view of improving the yield of the compound (32), it is preferred that the compound (21) is reacted with the compound (1) in a ratio of 1 molar equivalent or less to the latter compound to selectively form the compound (11) and then the compound (22) is reacted with the compound (11) in a ratio of 1 to 2 molar equivalents to the latter compound. When the amount of the compound (22) is less than 1 molar equivalent, the yield of the objective compound (32) decreases. When the amount is more than 2 molar equivalents, the compound (33) is formed by the ester exchange reaction between the formed compound (32) and the compound (22), so that the yield of the objective compound (32) decreases.

The amount of the catalyst in the case where the catalyst is used in the reaction is variously selected depending on the catalyst, but is preferably 0.01 to 30% by mass and, in consideration of the reaction activity and a catalyst removal step after the reaction, is more preferably 0.1 to 10% by mass based on the substrate.

The amount of the promoter in the case where the promoter is used in the reaction is variously selected depending on the promoter, but is preferably 0.01 to 30% by mass and, in consideration of the reaction activity and a promoter removal step after the reaction, is more preferably 0.1 to 10% by mass based on the substrate.

Since the compound (21) and the compound (22) mostly have a low compatibility with the compound (1) and the compound (11), the reaction sometimes forms a heterogeneous system at an early reaction stage. Accordingly, in the reaction, a solvent may be used for the purpose of promoting the reaction. However, when volume efficiency of a reactor and loss of the objective product at a solvent separation step are considered, it is preferred to carry out the reaction without any solvent, if possible.

The solvent may be one stably present at the reaction temperature and showing a high solubility of the starting materials and, in view of capability of separation of the compound (1), the compound (11), the compound (21), the compound (22), the compound (32), and by-products by distillation after the reaction, it is preferred to use a solvent having a boiling point different from that of each of these compounds or to use the compound (32) as the solvent.

As the solvent, carbonate compounds different in boiling point, the compound (32), ethers having a relatively high boiling point are preferred. Specific examples thereof include ethylene carbonate, propylene carbonate, dimethyl carbonate, diethyl carbonate, dipropyl carbonate, dibutyl carbonate, dioctyl carbonate, glyme, diglyme, triglyme and tetraglyme.

When the effect of using the solvent is considered, the amount of the solvent is preferably an amount so that the concentration of the substrate becomes 10 to 80% by mass. However, in the case of a substrate where the effect of using the solvent is not so much observed, no solvent (substrate concentration of 100% by mass) is preferred in view of separation.

In the invention, at least a part of the reaction between the compound (1) and the compound (21) and/or the compound (22) is preferably carried out at a reaction temperature of 40 to 200°C.

When the reaction temperature is lower than 40°C, the yield of the carbonate compound is extremely low. When the reaction temperature exceeds 200°C, decrease in yield owing to the decomposition of the compound (1) to be used as a starting material becomes remarkable. When the reaction temperature falls within the above range, the carbonate compound can be produced in high yields at a reaction rate capable of industrial use.

The reaction temperature is more preferably 40 to 160°C, more preferably 50 to 150°C, and particularly preferably 60 to 140°C.

The efficiency of the reaction can be improved by carrying out the reaction at different reaction temperatures at the early reaction stage and at the later reaction stage. Namely, the reaction of forming the compound (11) by reacting the compound (21) with the compound (1) is preferably at a reaction temperature of 40°C or lower in view of improving the yield of the compound (11). The reaction can be carried out at a temperature higher than 40°C but since the reaction is too vigorous, by-products may be increased or the compound (31) that is a disubstituted product may be formed, thereby the yield of the objective product being lowered in some cases. In the case where the compound (1) is reacted with the compound (21) at the reaction temperature of 40°C or lower, the compound (11) can be selectively synthesized even when the compound (21) is reacted with the compound (1) in a ratio of 1 equivalent or more to the latter compound. However, unless the reaction is carried out after unreacted compound (21) is removed from the reaction system before the next compound (22) is reacted, the lowering of the yield of the objective compound (32) may be caused through the production of the compound (31) as a by-product.

The reaction between the compound (11) and the compound (22) is preferably carried out at a reaction temperature of 40 to 200°C, and more preferably carried out at a reaction temperature of 50 to 200°C.

Thus, since the difference between the reaction rate of the first step and the reaction rate of the second step is large, there are advantages that the compound (11) as an intermediate can be easily synthesized and isolated and the asymmetrical compound (22), which is hitherto hardly synthesized, can be selectively synthesized utilizing the difference between the reaction rates.

The reaction pressure is usually atmospheric pressure. Depending on the vapor pressure of the compound (21) and the compound (22) at the reaction temperature, it is preferred to apply pressure.

In the present reaction, CHX¹X²X³ and/or CHX⁴X⁵X⁶ (chloroform and the like), which are halogenated methanes having a low boiling point, are formed as the reaction proceeds. Accordingly, in order to improve the reaction yield by shifting the reaction equilibrium to the compound (31) side and to complete the reaction stoichiometrically, it is preferred to carry out the reaction with removing the formed CHX¹X²X³ and/or CHX⁴X⁵X⁶ from the reaction system by distillation.

As a method for removing halogenated methanes by distillation, a reaction distillation mode utilizing the fact that the halogenated methanes each has a low boiling point as compared with the compound (21), the compound (11), the compound (22) and the compound (32) is preferred from the viewpoint of easy implementation.

### (Process for producing compound (3a), compound (3b))

The compound (3a) and the compound (3b) are produced by reacting the compound (1) with a compound (23), optionally, in the presence of a catalyst.

[Chem. 14]

**HO-R³-OH** (23)

Examples of the compound (23) include divalent fluorine-containing aliphatic alcohols and divalent fluorine-containing phenols.

The divalent fluorine-containing aliphatic alcohol is preferably a polyfluoroalkanediol having 3 to 64 carbon atoms, more preferably a polyfluoroalkanediol having 3 to 14 carbon atoms, most preferably a polyfluoroalkanediol having 3 to 10 carbon atoms, which has no fluorine atom at the α-position and may have an etheric oxygen atom, in view of versatility on industrial use.

The divalent fluorine-containing aliphatic alcohol is, in view of usefulness of the compound (3a) and the compound (3b), preferably 3,3,3-trifluoro-1,2-propanediol, 4,4,4,3,3-pentafluoro-1,2-butanediol, 1,1,1,4,4,4-hexafluoro-2,3-butanediol, 3,3,4,4-tetrafluoro-1,6-hexanediol, 2,2,3,3,4,4,5,5-octafluoro-1,6-hexanediol, 3,3,4,4,5,5,6,6-octafluoro-1,8-octanediol, or a compound represented by the following formula (X):

HO-CH₂CF₂-(CF₂CF₂O)ₘ-CF₂CH₂-OH (X)

wherein m is an integer of 2 to 30.

Examples of the divalent fluorine-containing phenols include tetrafluorohydroquinone, tetrafluororesorcinol and 2,2-bis(4-hydroxyphenyl)-hexafluoropropane [bisphenol AF], and 2,2-bis(4-hydroxyphenyl)hexafluoropropane, tetrafluorohydroquinone or tetrafluororesorcinol is preferred.

In the case where the objective product is the compound (3a), with regard to the ratio of the substrates (starting materials), the ratio of the compound (23) is preferably 0.1 to 10 molar equivalents to the compound (1) and, in view of the reaction efficiency and the yield, is more preferably 0.5 to 2 molar equivalents.

In the case where the objective product is the compound (3b), the ratio of the substrates varies depending on the molecular weight of the compound (3b) but the ratio of the compound (23) is preferably 0.5 to 2 molar equivalents to the compound (1) and, in view of the reaction efficiency and the yield, is more preferably 0.75 to 1.5 molar equivalents.

The amount of the catalyst in the case where the catalyst is used in the reaction is variously selected depending on the catalyst, but is preferably 0.01 to 30% by mass and, in consideration of reactivity and a catalyst removal step after the reaction, is more preferably 0.1 to 10% by mass based on the substrate.

The amount of the promoter in the case where the promoter is used in the reaction is variously selected depending on the promoter, but is preferably 0.01 to 30% by mass and, in consideration of reactivity and a promoter removal step after the reaction, is more preferably 0.1 to 10% by mass based on the substrate.

Since the compound (23) mostly has a low compatibility with the compound (1), the reaction sometimes forms a heterogeneous system at an early reaction stage. Accordingly, in the reaction, a solvent may be used for the purpose of promoting the reaction. However, when volume efficiency of a reactor and loss of the objective product at a solvent separation step are considered, it is preferred to carry out the reaction without any solvent, if possible.

The solvent may be one stably present at the reaction temperature and showing a high solubility of the starting materials and, in view of capability of separation of the compound (1), the compound (23), the compound (3a), the compound (3b), and by-products by distillation after the reaction, it is preferred to use a solvent having a boiling point different from that of each of these compounds or to use the compound (3a) as the solvent.

As the solvent, carbonate compounds different in boiling point, the compound (3 a), ethers having a relatively high boiling point are preferred. Specific examples thereof include ethylene carbonate, propylene carbonate, dimethyl carbonate, diethyl carbonate, dipropyl carbonate, dibutyl carbonate, dioctyl carbonate, glyme, diglyme, triglyme and tetraglyme.

When the effect of using the solvent is considered, the amount of the solvent is preferably an amount so that the concentration of the substrate becomes 10 to 80% by mass. However, in the case of a substrate where the effect of using the solvent is not so much observed, no solvent (substrate concentration of 100% by mass) is preferred in view of separation.

The reaction temperature varies depending on the substrates, catalysts, and the like and is usually 0 to 200°C.

The efficiency of the reaction can be improved by carrying out the reaction at different reaction temperatures at the early reaction stage and at the later reaction stage. This is because the substitution reactions of the two functional groups in the compound (1) proceeds stepwise and the reaction rate of the first step substitution reaction is high but the reaction rate of the second substitution reaction is comparatively low. Since the first step substitution reaction easily proceeds at a relatively low temperature of about 0 to 100°C and is a reaction with severe heat generation for a while, the reaction is preferably allowed to proceed at a relatively low temperature at the early reaction stage. The second step substitution reaction is carried out at a relatively high temperature of about 50 to 200°C in view of the reaction rate.

In this connection, in the case where the objective product has a stable 5-membered ring structure, such as an alkyl-substituted ethylene carbonate, since a stabilization effect by cyclization is large, the reaction of the second step between the compound (1) and the compound (23) also proceeds at a very high reaction rate and the reaction is completed within a short period of time even at a relatively low temperature of 0 to 80°C.

The reaction pressure is usually atmospheric pressure. Depending on the vapor pressure of the compound (23) at the reaction temperature, it is preferred to apply pressure.

In the present reaction, CHX¹X²X³ and/or CHX⁴X⁵X⁶ (chloroform and the like), which are halogenated methanes having a low boiling point, are formed as the reaction proceeds. Accordingly, in order to improve the reaction yield by shifting the reaction equilibrium to the compound (3a) and compound (3b) side and to complete the reaction stoichiometrically, it is preferred to carry out the reaction with removing the formed CHX¹X²X³ and/or CHX⁴X⁵X⁶ from the reaction system by distillation.

As a method for removing halogenated methanes by distillation, a reaction distillation mode utilizing the fact that the halogenated methanes each has a low boiling point as compared with the compound (23), the compound (3a), the compound (3b), and the compound (31) is preferred from the viewpoint of easy implementation.

### (Process for producing branched carbonate compound)

The branched carbonate compound is produced by reacting the compound (1) with a fluorine-containing compound having more than two OH groups.

Examples of the fluorine-containing compound having more than two OH groups include trivalent or higher valent fluorine-containing aliphatic alcohols, trivalent or higher valent fluorine-containing phenols, and mixtures of them and the above fluorine-containing compound having two OH groups. In the case of the mixtures, the average value of the terminal OH groups is taken as the number of OH groups.

Examples of the trivalent or higher valent fluorine-containing aliphatic alcohols include compounds obtained by replacing a part or all of the hydrogen atom(s) of the following compound by fluorine atom(s): glycerin, diglycerin, polyglycerin, trimethylolpropane, 1,2,6-hexanetriol, pentaerythritol, tetramethylolcyclohexane, methylglycoside, sorbitol, mannitol, dulcitol, sucrose, etc.

Examples of the trivalent or higher valent fluorine-containing phenols include compounds obtained by replacing a part or all of the hydrogen atom(s) of fluoroglycinol by fluorine atom(s), compounds obtained by replacing a part or all of the hydrogen atom(s) of condensates of phenols by fluorine atom(s).

Examples of the condensates of phenols include resol-type initial condensates wherein phenols are condensed and combined with excess of aldehydes in the presence of an alkali catalyst; benzylic-type initial condensates which are produced by the reaction in a non-aqueous system at the time when the resol-type initial condensates are synthesized; and novolak-type initial condensates wherein excess of phenols are reacted with formaldehydes in the presence of an acid catalyst. The molecular weight of the initial condensates is preferably about 200 to 10000.

The ratio of the substrates varies depending on the molecular weight of the branched carbonate compound but the ratio of the compound having more than two OH groups is preferably 0.5 to 2 molar equivalents to the compound (1) and more preferably 0.75 to 1.5 molar equivalents.

The amount of the catalyst in the case where the catalyst is used in the reaction is variously selected depending on the catalyst, but is preferably 0.01 to 30% by mass and, in consideration of reactivity and a catalyst removal step after the reaction, is more preferably 0.1 to 10% by mass based on the substrate.

The amount of the promoter in the case where the promoter is used in the reaction is variously selected depending on the promoter, but is preferably 0.01 to 30% by mass and, in consideration of reactivity and a promoter removal step after the reaction, is more preferably 0.1 to 10% by mass based on the substrate.

Since the fluorine-containing compound having more than two OH groups mostly has a low compatibility with the compound (1), the reaction sometimes forms a heterogeneous system at an early reaction stage. Accordingly, in the reaction, a solvent may be used for the purpose of promoting the reaction. However, when volume efficiency of a reactor and loss of the objective product at a solvent separation step are considered, it is preferred to carry out the reaction without any solvent, if possible.

The solvent may be one stably present at the reaction temperature and showing a high solubility of the starting materials and, in view of capability of separation of the compound (1), the fluorine-containing compound having more than two OH groups, the branched carbonate compound, and by-products by distillation after the reaction, it is preferred to use a solvent having a boiling point different from that of each of these compounds or to use the compound (3a) as the solvent.

As the solvent, carbonate compounds different in boiling point, the compound (3a), ethers having a relatively high boiling point are preferred. Specific examples thereof include ethylene carbonate, propylene carbonate, dimethyl carbonate, diethyl carbonate, dipropyl carbonate, dibutyl carbonate, dioctyl carbonate, glyme, diglyme, triglyme and tetraglyme.

When the effect of using the solvent is considered, the amount of the solvent is preferably an amount so that the concentration of the substrate becomes 10 to 80% by mass. However, in the case of a substrate where the effect of using the solvent is not so much observed, no solvent (substrate concentration of 100% by mass) is preferred in view of separation.

The reaction temperature varies depending on the substrates, catalysts, and the like and is usually 0 to 200°C.

The efficiency of the reaction can be improved by carrying out the reaction at different reaction temperatures at the early reaction stage and at the later reaction stage. This is because the substitution reactions of the two functional groups in the compound (1) proceeds stepwise and the reaction rate of the first step substitution reaction is high but the reaction rate of the second substitution reaction is comparatively low. Since the first step substitution reaction easily proceeds at a relatively low temperature of about 0 to 100°C and is a reaction with severe heat generation for a while, the reaction is preferably allowed to proceed at a relatively low temperature at the early reaction stage. The second step substitution reaction is carried out at a relatively high temperature of about 50 to 200°C in view of the reaction rate.

The reaction pressure is usually atmospheric pressure. Depending on the vapor pressure of the compound having more than two OH groups at the reaction temperature, it is preferred to apply pressure.

In the present reaction, chloroform, which is a halogenated methane having a low boiling point, is formed as the reaction proceeds. Accordingly, in order to improve the reaction yield by shifting the reaction equilibrium to the branched carbonate compound side and to complete the reaction stoichiometrically, it is preferred to carry out the reaction with removing the formed chloroform from the reaction system by distillation.

As a method for removing the halogenated methanes by distillation, a reaction distillation mode utilizing the fact that the halogenated methanes each has a low boiling point as compared with the fluorine-containing compound having more than two OH groups and the branched carbonate compound is preferred from the viewpoint of easy implementation.

Since the process for producing a carbonate compound of the invention as described in the above is a process wherein the compound (1) is reacted with the fluorine-containing compound having one OH group to obtain a carbonate compound, a symmetrical di(fluoroalkyl) carbonate or di(fluoroaryl) carbonate and an asymmetrical di(fluoroalkyl) carbonate or di(fluoroaryl) carbonate can be selectively prepared without any inhibition in high yields by suitably changing the fluorine-containing compound having one OH group in one reaction process.

Moreover, since the process for producing a carbonate compound of the invention is a process wherein the compound (1) is reacted with the fluorine-containing compound having two or more OH groups to obtain a carbonate compound, a fluorine-containing cyclic carbonate and a fluorine-containing polycarbonate can be selectively prepared without any inhibition in high yields by suitably changing the fluorine-containing compound having two or more OH groups in one reaction process.

Furthermore, since the by-product is an organic compound having a low boiling point, such as chloroform, a production process can be simplified, for example, by-products can be easily removed from the reaction system, unlike other methods such as a method using phosgene.

Moreover, by changing the compound (I) to hexachloroacetone, industrially useful chloroform can be simultaneously produced.

Furthermore, by the use of a partially fluorinated compound as the compound (I), industrially useful dichlorofluoromethane (R21), chlorodifluoromethane (R22), or the like can be simultaneously produced.

### EXAMPLES

The present invention will be illustrated in greater detail with reference to the following Examples, but the invention should not be construed as being limited thereby.

Examples 1 to 13 are Invention Examples.

### (Gas chromatograph)

The analysis on a chromatograph (hereinafter referred to as GC) was performed using a 6890 series manufactured by Agilent Company.

### Example 1

After 262 g (0.99 mol) of hexachloroacetone, 392 g (2.97 mol) of 2,2,3,3-tetrafluoro-1-propanol, and 4 g of KF (potassium fluoride) were charged into a 500 mL glass reactor fitted with a stirrer, a reflux condenser at 20°C, and a distillation line, the temperature was gradually elevated under stirring and a reaction was carried out at an inner temperature of 100°C. While chloroform formed by the reaction was removed by distillation through the distillation line, the reaction was carried out for 10 hours. After the reaction was completed, fractions distilled from the distillation line and a reaction crude liquid present in the reactor were recovered to obtain 645 g of a recovered crude liquid (recovery rate: 98%). As a result of analysis on GC of an organic component recovered by simple distillation of the recovered crude liquid under vacuum, it was confirmed that compounds shown in Table 1 were formed in yields shown in Table 1.

From the results shown in Table 1, the conversion rate of hexachloroacetone was 100%, the yield of (CHF₂CF₂CH₂O)₂C(=O) based on hexachloroacetone was 93%, and the yield of chloroform was 96%.

**[Table 1]**

| Compound | GC Composition (% by mass) | (Yield |
|---|---|---|
| Hexachloroacetone | 0% | 0 g |
| 2,2,3,3-Tetrafluoro-1-propanol | 20.9% | 134.1 g |
| Chloroform | 35.3% | 226 g |
| Carbon tetrachloride | 0.03% | 0.2 g |
| (CHF₂CF₂CH₃O)₂C(=O) | 41.6% | 267 g |
| CHF₂CF₂CH₂OC(=O)CCl₃ | 2.1% | 13.3 g |
| Others | 0.07% | 0.4 g |

### Example 2

After 262 g (0.99 mol) of hexachloroacetone, 392 g (2.97 mol) of 2,2,3,3-tetrafluoro-1-propanol, and 4 g of tetrabutylammonium bromide (hereinafter referred to as TBAB) were charged into a reactor similar to that of Example 1, the temperature was gradually elevated under stirring and a reaction was carried out at an inner temperature of 100°C. While chloroform formed by the reaction was removed by distillation through the distillation line, the reaction was carried out for 20 hours. After the reaction was completed, fractions distilled from the distillation line and a reaction crude liquid present in the reactor were recovered to obtain 625 g of a recovered crude liquid (recovery rate: 95%). As a result of analysis on GC of an organic component recovered by simple distillation of the recovered crude liquid under vacuum, it was confirmed that compounds shown in Table 2 were formed in yields shown in Table 2.

From the results shown in Table 2, the conversion rate of hexachloroacetone was 100%, the yield of (CHF₂CF₂CH₂O)₂C(=O) based on hexachloroacetone was 89%, and the yield of chloroform was 90%.

**[Table 2]**

| Compound | GC Composition (% by mass) | Yield |
|---|---|---|
| Hexachloroacetone | 0% | 0 g |
| 2,2,3,3-Tetrafluoro-1-propanol | 20.2% | 125.2 g |
| Chloroform | 34.2% | 212.5 g |
| Carbon tetrachloride | 0.03% | 0.2 g |
| (CHF₂CF₂CH₂O)₂C(=O) | 41.2% | 255.8 g |
| CHF₂CF₂CH₂OC(=O)CCl₃ | 4.3% | 26.9 g |
| Others | 0.07% | 0.4 g |

### Example 3

After 262 g (0.99 mol) of hexachloroacetone, 45.5 g (0.99 mol) of ethanol, and 4 g of KF were charged into a reactor similar to that of Example 1, the whole was stirred under stirring at 30°C for 1 hour. Then 130.7 g (0.99 mol) of 2,2,3,3-tetrafluoro-1-propanol was added thereto, the temperature was gradually elevated, and a reaction was carried out at an inner temperature of 100°C. While chloroform formed by the reaction was removed by distillation through the distillation line, the reaction was carried out for 10 hours. After the reaction was completed, fractions distilled from the distillation line and a reaction crude liquid present in the reactor were recovered to obtain 431.1 g of a recovered crude liquid (recovery rate: 97.5%). As a result of analysis on GC of an organic component recovered by simple distillation of the recovered crude liquid under vacuum, it was confirmed that compounds shown in Table 3 were formed in yields shown in Table 3.

From the results shown in Table 3, the conversion rate of hexachloroacetone was 100%, the yield of CHF₂CF₂CH₂OC(=O)CH₂CH₃ based on hexachloroacetone was 74%, and the yield of chloroform was 93%.

**[Table 3]**

| Compound | GC Composition (% by mass) | Yield |
|---|---|---|
| Hexachloroacetone | 0% | 0 g |
| Ethanol | 10% | 0 g |
| 2,2,3,3-Tetrafluoro-1-propanol | 2.9% | 12.4 g |
| Chloroform | 51.7% | 220.9 g |
| Carbon tetrachloride | 0.1% | 0.4 g |
| CHF₂CF₂CH₂OC(=O)CH₂CH₃ | 35.0% | 149.6 g |
| Others | 10.3% | 43.8 g |

### Example 4

After 262 g (0.99 mol) of hexachloroacetone, 392 g (2.97 mol) of 2,2,3,3-tetrafluoro-1-propanol, and 4 g of CsF were charged into a reactor similar to that of Example 1, the temperature was gradually elevated under stirring and a reaction was carried out at an inner temperature of 100°C. While chloroform formed by the reaction was removed by distillation through the distillation line, the reaction was carried out for 20 hours. After the reaction was completed, fractions distilled from the distillation line and a reaction crude liquid present in the reactor were recovered to obtain 634 g of a recovered crude liquid (recovery rate: 96%). As a result of analysis on GC of an organic component recovered by simple distillation of the recovered crude liquid under vacuum, it was confirmed that compounds shown in Table 4 were formed in yields shown in Table 4.

From the results shown in Table 4, the conversion rate of hexachloroacetone was 100%, the yield of (CHF₂CF₂CH₂O)₂C(=O) based on hexachloroacetone was 93%, and the yield of chloroform was 92%.

**[Table 4]**

| Compound | GC Composition (% by mass) | Yield |
|---|---|---|
| Hexachloroacetone | 0% | 0 g |
| 2,2,3,3-Tetrafluoro-1-propanol | 19.9% | 125.2 g |
| Chloroform | 34.5% | 217.5 g |
| Carbon tetrachloride | 0.03% | 0.2 g |
| (CHF₂CF₂CH₂O)₂C(=O) | 42.2% | 265.8 g |
| CHF₂CF₂CH₂OC(=O)CCl₃ | 2.7% | 16.9 g |
| Others | 0.67% | 4.4 g |

### Example 5

After 262 g (0.99 mol) of hexachloroacetone, 262 g (1.98 mol) of 2,2,3,3-tetrafluoro-1-propanol, 2 g of KF, and 2 g of cerium oxide (CeO/Ce₂O₃: manufactured by Daiichi Kigenso Kagaku Kogyo Co., Ltd.) were charged into a reactor similar to that of Example 1, the temperature was gradually elevated under stirring and a reaction was carried out at an inner temperature of 100°C. While chloroform formed by the reaction was removed by distillation through the distillation line, the reaction was carried out for 20 hours. After the reaction was completed, fractions distilled from the distillation line and a reaction crude liquid present in the reactor were recovered to obtain 517 g of a recovered crude liquid (recovery rate: 98%). As a result of analysis on GC of an organic component recovered by simple distillation of the recovered crude liquid under vacuum, it was confirmed that compounds shown in Table 5 were formed in yields shown in Table 5.

From the results shown in Table 5, the conversion rate of hexachloroacetone was 100%, the yield of (CHF₂CF₂CH₂O)₂C(=O) based on hexachloroacetone was 93%, and the yield of chloroform was 94%.

**[Table 5]**

| Compound | GC Composition (% by mass) | Yield |
|---|---|---|
| Hexachloroacetone | 0% | 0 g |
| 2,2,3,3-Tetrafluoro-1-propanol | 1.3% | 6.5 g |
| Chloroform | 43.2% | 221.8 g |
| Carbon tetrachloride | 0.03% | 0.2 g |
| (CHF₂CF₂CH₂O)₂C(=O) | 52.3% | 268.0 g |
| CHF₂CF₂CH₂OC(=O)CCl₃ | 2.4% | 12.5 g |
| Others | 10.77% | 4.0 g |

### Example 6

After 262 g (0.99 mol) of hexachloroacetone, 262 g (1.98 mol) of 2,2,3,3-tetrafluoro-1-propanol, 2 g of KF, and 2 g of silica-alumina (SiO₂·Al₂O₃: manufactured by Nikki Chemical Co., Ltd.) were charged into a reactor similar to that of Example 1, the temperature was gradually elevated under stirring and a reaction was carried out at an inner temperature of 100°C. While chloroform formed by the reaction was removed by distillation through the distillation line, the reaction was carried out for 20 hours. After the reaction was completed, fractions distilled from the distillation line and a reaction crude liquid present in the reactor were recovered to obtain 518 g of a recovered crude liquid (recovery rate: 98%). As a result of analysis on GC of an organic component recovered by simple distillation of the recovered crude liquid under vacuum, it was confirmed that compounds shown in Table 6 were formed in yields shown in Table 6.

From the results shown in Table 6, the conversion rate of hexachloroacetone was 100%, the yield of (CHF₂CF₂CH₂O)₂C(=O) based on hexachloroacetone was 94%, and the yield of chloroform was 96%.

**[Table 6]**

| Compound | GC Composition (% by mass) | Yield |
|---|---|---|
| Hexachloroacetone | 0% | 0 g |
| 2,2,3,3-Tetrafluoro-1-propanol | 0.7% | 3.6 g |
| Chloroform | 44.1% | 226.6 g |
| Carbon tetrachloride | 0.04% | 0.2 g |
| (CHF₂CF₂CH₂O)₂C(=O) | 52.7% | 271.0 g |
| CHF₂CF₂CH₂OC(=O)CCl₃ | 1.5% | 7.5 g |
| Others | 0.96% | 5.1 g |

### Example 7

After 262 g (0.99 mol) of hexachloroacetone, 262 g (1.98 mol) of 2,2,3,3-tetrafluoro-1-propanol, 2 g of KF, and 2 g of ZnO·ZrO₂ (manufactured by Daiichi Kigenso Kagaku Kogyo Co., Ltd.) were charged into a reactor similar to that of Example 1, the temperature was gradually elevated under stirring and a reaction was carried out at an inner temperature of 100°C. While chloroform formed by the reaction was removed by distillation through the distillation line, the reaction was carried out for 20 hours. After the reaction was completed, fractions distilled from the distillation line and a reaction crude liquid present in the reactor were recovered to obtain 518 g of a recovered crude liquid (recovery rate: 98%). As a result of analysis on GC of an organic component recovered by simple distillation of the recovered crude liquid under vacuum, it was confirmed that compounds shown in Table 7 were formed in yields shown in Table 7.

From the results shown in Table 7, the conversion rate of hexachloroacetone was 100%, the yield of (CHF₂CF₂CH₂O)₂C(=O) based on hexachloroacetone was 95%, and the yield of chloroform was 96%.

**[Table 7]**

| Compound | GC Composition (% by mass) | Yield |
|---|---|---|
| Hexachloroacetone | 0% | 0 g |
| 2,2,3,3-Tetrafluoro-1-propanol | 0.6% | 3.3 g |
| Chloroform | 44.2% | 227.2 g |
| Carbon tetrachloride | 0.04% | 0.2 g |
| (CHF₂CF₂CH₂O)₂C(=O) | 52.9% | 272.0 g |
| CHF₂CF₂CH₂OC(=O)CCl₃ | 1.4% | 7.0 g |
| Others | 0.86% | 4.3 g |

### Example 8

After 262 g (0.99 mol) of hexachloroacetone, 262 g (1.98 mol) of 2,2,3,3-tetrafluoro-1-propanol, 2 g of KF, and 2 g of zirconia (ZrO₂, manufactured by Daiichi Kigenso Kagaku Kogyo Co., Ltd.) were charged into a 500 mL pressure tight reactor made of Hastelloy, the temperature was gradually elevated under stirring and a reaction was carried out at an inner temperature of 140°C for 10 hours. After the reaction was completed, 527 g of a recovered crude liquid present in the reactor was recovered (recovery rate: 99.8%). As a result of analysis on GC of an organic component recovered by simple distillation of the recovered crude liquid under vacuum, it was confirmed that compounds shown in Table 8 were formed in yields shown in Table 8.

From the results shown in Table 8, the conversion rate of hexachloroacetone was 100%, the yield of (CHF₂CF₂CH₂O)₂C(=O) based on hexachloroacetone was 99%, and the yield of chloroform was 99%.

**[Table 8]**

| Compound | GC Composition (% by mass) | Yield |
|---|---|---|
| Hexachloroacetone | 0% | 0 g |
| 2,2,3,3-Tetrafluoro-1-propanol | 0.2% | 1.1 g |
| Chloroform | 44.9% | 235 g |
| Carbon tetrachloride | 0.04% | 0.2 g |
| (CHF₂CF₂CH₂O)₂C(=O) | 54.3% | 284 g |
| CHF₂CF₂CH₂OC(=O)CCl₃ | 0.4% | 2.3 g |
| Others . | 0.16% | 0.4 g |

### Example 9

After 262 g (0.99 mol) of hexachloroacetone, 198 g (1.98 mol) of 2,2,2-trifluoroethanol, 2 g of KF, and 2 g of zirconia (ZrO₂, manufactured by Daiichi Kigenso Kagaku Kogyo Co., Ltd.) were charged into a 500 mL pressure tight reactor made of Hastelloy, the temperature was gradually elevated under stirring and a reaction was carried out at an inner temperature of 140°C for 10 hours. After the reaction was completed, 462 g of a recovered crude liquid present in the reactor was recovered (recovery rate: 99.6%). As a result of analysis on GC of an organic component recovered by simple distillation of the recovered crude liquid under vacuum, it was confirmed that compounds shown in Table 9 were formed in yields shown in Table 9.

From the results shown in Table 9, the conversion rate of hexachloroacetone was 100%, the yield of (CF₃CH₂O)₂C(=O) based on hexachloroacetone was 99%, and the yield of chloroform was 99%.

**[Table 9]**

| Compound | GC Composition (% by mass) | Yield |
|---|---|---|
| Hexachloroacetone | 0% | 0 g |
| 2,2,2-Trifluoroethanol | 0.12% | 0.6 g |
| Chloroform | 51.1% | 234 g |
| Carbon tetrachloride | 0.04% | 0.2 g |
| (CF₃CH₂O)₂C(=O) | 48.3% | 221 g |
| CF₃CH₂OC(=O)CCl₃ | 0.3% | 1.4 g |
| Others | 0.14% | 0.8 g |

### Example 10

After 262 g (0.99 mol) of hexachloroacetone, 333 g (1.98 mol) of 1,1,1,3,3,3-hexafluoroisopropanol, 4 g of KF, and 4 g of silica-alumina (SiO₂·Al₂O₃: manufactured by Nikki Chemical Co., Ltd.) were charged into a 500 mL pressure tight reactor made of Hastelloy, the temperature was gradually elevated under stirring and a reaction was carried out at an inner temperature of 140°C for 20 hours. After the reaction was completed, 599 g of a recovered crude liquid present in the reactor was recovered (recovery rate: 99.4%). As a result of analysis on GC of an organic component recovered by simple distillation of the recovered crude liquid under vacuum, it was confirmed that compounds shown in Table 10 were formed in yields shown in Table 10.

From the results shown in Table 10, the conversion rate of hexachloroacetone was 50%, the yield of ((CF₃)₂CHO)₂C(=O) based on hexachloroacetone was 15%, and the yield of chloroform was 32%.

**[Table 10]**

| Compound | GC Composition (% by mass) | Yield |
|---|---|---|
| Hexachloroacetone | 22.0% | 130 g |
| 1,1,1,3,3,3-Hexafluoroisopropanol | 38.1% | 225 g |
| Chloroform | 12.8% | 76 g |
| Carbon tetrachloride | 0.08% | 0.5 g |
| ((CF₃)₂CHO)₂C(=O) | 9.0% | 53 g |
| (CF₃)₂CHOC(=O)CCl₃ | 17.9% | 106 g |
| Others | 0.12% | 0.5 g |

### Example 11

Into a 500 mL glass reactor fitted with a stirrer, a reflux condenser at 20°C, and a distillation line was charged 4 g ofNaH, and then 262 g (1.98 mol) of 2,2,3,3-tetrafluoro-1-propanol was slowly added dropwise over a period of 30 minutes. After completion of the dropwise addition, 262 g (0.99 mol) of hexachloroacetone was added dropwise under cooling with a water bath so that the inner temperature did not reach 50°C or higher. After completion of the dropwise addition, the temperature was gradually elevated under stirring and a reaction was carried out at an inner temperature of 100°C for 10 hours. After the reaction was completed, 517 g of a recovered crude liquid present in the reactor was recovered (recovery rate: 98.0%). As a result of analysis on GC of an organic component recovered by simple distillation of the recovered crude liquid under vacuum, it was confirmed that compounds shown in Table 11 were formed in yields shown in Table 11.

From the results shown in Table 11, the conversion rate of hexachloroacetone was 100%, the yield of (CHF₂CF₂CH₂O)₂C(=O) based on hexachloroacetone was 2%, and the yield of chloroform was 49%.

**[Table 11]**

| Compound | GC Composition (% by mass) | Yield |
|---|---|---|
| Hexachloroacetone | 0% | 0 g |
| 2,2,3,3 -tetrafluoro-1-propanol | 24.7% | 126 g |
| Chloroform | 22.9% | 117 g |
| Carbon tetrachloride | 0.06% | 0.3 g |
| (CHF₂CF₂CH₂O)₂C(=O) | 1.1% | 5.6 g |
| CHF₂CF₂CH₂OC(=O)CCl₃ | 51.1% | 261 g |
| Others | 0.14% | 1.1 g |

### Example 12

Into a 500 mL glass reactor fitted with a stirrer, a reflux condenser at 20°C, and a distillation line was charged 4 g of Na, and then 262 g (1.98 mol) of 2,2,3,3-tetrafluoro-1-propanol was slowly added dropwise over a period of 30 minutes. After completion of the dropwise addition, 262 g (0.99 mol) of hexachloroacetone was added dropwise under cooling with a water bath so that the inner temperature did not reach 50°C or higher. After completion of the dropwise addition, the temperature was gradually elevated under stirring and a reaction was carried out at an inner temperature of 100°C for 10 hours. After the reaction was completed, 516 g of a recovered crude liquid present in the reactor was recovered (recovery rate: 98.0%). As a result of analysis on GC of an organic component recovered by simple distillation of the recovered crude liquid under vacuum, it was confirmed that compounds shown in Table 12 were formed in yields shown in Table 12.

From the results shown in Table 12, the conversion rate of hexachloroacetone was 100%, the yield of (CHF₂CF₂CH₂O)₂C(=O) based on hexachloroacetone was 2%, and the yield of chloroform was 50%.

**[Table 12]**

| Compound | GC Composition (% by mass) | Yield |
|---|---|---|
| Hexachloroacetone | 0% | 0 g |
| 2,2,3,3-tetrafluoro-1-propanol | 24.3% | 124 g |
| Chloroform | 23.3% | 119 g |
| Carbon tetrachloride | 0.06% | 0.3 g |
| (CHF₂CF₂CH₂O)₂C(=O) | 1.23% | 6.3 g |
| CHF₂CF₂CH₂OC(=O)CCl₃ | 50.9% | 260 g |
| Others | 0.21% | 1.4 g |

### Example 13

After 262 g (0.99 mol) of hexachloroacetone, 262 g (1.98 mol) of 2,2,3,3-tetrafluoro-1-propanol, 2 g of KF, and 2 g of an anion type ion-exchange resin (Amberlyte IRA-900, Cl-form manufactured by Rohm and Haas Company) were charged into a reactor similar to that of Example 1, the temperature was gradually elevated under stirring and a reaction was carried out at an inner temperature of 100°C. While chloroform formed by the reaction was removed by distillation through the distillation line, the reaction was carried out for 20 hours. After the reaction was completed, fractions distilled from the distillation line and a reaction crude liquid present in the reactor were recovered to obtain 523 g of a recovered crude liquid (recovery rate: 99%). As a result of analysis on GC of an organic component recovered by simple distillation of the recovered crude liquid under vacuum, it was confirmed that compounds shown in Table 13 were formed in yields shown in Table 13.

From the results shown in Table 13, the conversion rate of hexachloroacetone was 100%, the yield of (CHF₂CF₂CH₂O)₂C(=O) based on hexachloroacetone was 97%, and the yield of chloroform was 98%.

**[Table 13]**

| Compound | GC Composition (% by mass) | Yield |
|---|---|---|
| Hexachloroacetone | 0% | 0 g |
| 2,2,3,3-tetrafluoro-1-propanol | 0.5% | 2.6 g |
| Chloroform | 44.6% | 231.3 g |
| Carbon tetrachloride | 0.05% | 0.3 g |
| (CHF₂CF₂CH₂O)₂C(=O) | 53.8% | 279.0 g |
| CHF₂CF₂CH₂OC(=O)CCl₃ | 1.0% | 5.4 g |
| Others | 0.05% | 0.4 g |

This application is based on Japanese Patent Application No. 2007-312655 filed December 3, 2007, Japanese Patent Application No. 2007-321773 filed December 13, 2007 and Japanese Patent Application No. 2008-208727 filed August 13, 2008.

### INDUSTRIAL APPLICABILITY

The fluorine-containing carbonate compounds obtained by the production process of the invention can be applied to various uses and are useful as organic solvents, resin raw materials, raw materials for pharmaceuticals and agricultural chemicals, and the like. Moreover, the fluorine-containing aromatic carbonate compounds are also useful as heat-resistant media.

In particular, the fluorine-containing cyclic carbonates obtained by the production process of the invention are industrially extremely useful as solvent applicable to various uses, electrolytes, resist removers, acrylic fiber processors, hydroxyethylating agents, raw materials for pharmaceuticals, soil hardeners, and the like.

Moreover, the fluorine-containing polycarbonates obtained by the production process of the invention are useful, as oligomers having a reactive OH group in the terminal, as raw materials for various polymer materials such as highly functional polyurethanes, polyesters, polycarbonates, and epoxy resins, reactive diluents, reactive plasticizers, and the like.

## Claims

1. A process for producing a carbonate compound comprising reacting a compound represented by the following formula (1) with a fluorine-containing compound having at least one OH group in the presence of a catalyst to obtain a fluorine-containing compound having a carbonate bond: 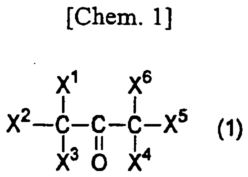 wherein X¹ to X⁶ each represents a chlorine atom, and
wherein the fluorine-containing compound having at least one OH group is a polyfluoroalkanemonool having 2 to 10 carbon atoms which has no fluorine atom at the α-position and may have an etheric oxygen atom, or a polyfluoroalkanediol having 3 to 10 carbon atoms which has no fluorine atom at the α-position and may have an etheric oxygen atom.

2. The process for producing a carbonate compound according to claim 1, wherein the catalyst comprises a halogen salt.

3. The process for producing a carbonate compound according to claim 2, wherein the halogen salt comprises one or more member selected from the group consisting of halogen salts of alkali metals, halogen salts of alkali earth metals, halogen salts of ammoniums, halogen salts of quaternary ammoniums, and ion-exchange resins having a halogen salt structure.

4. The process for producing a carbonate compound according to claim 2 or 3, wherein the halogen salt is a fluoride of an alkali metal.

5. The process for producing a carbonate compound according to any one of claims 1 to 4, wherein the reaction is carried out in the presence of the catalyst and a promoter, wherein the promoter is a solid acid catalyst.

6. The process for producing a carbonate compound according to claim 5, wherein the solid acid catalyst comprises at least one member selected from the group consisting of metal oxides having a strong acid point, heteropoly acids, and cation-exchange resins.

7. The process for producing a carbonate compound according to claim 6, wherein the metal oxides having a strong acid point comprise at least one member selected from the group consisting of cerium oxide (CeO₂/Ce₂O₃), silica-alumina (SiO₂·Al₂O₃), γ-alumina (Al₂O₃), silica-magnesia (SiO₂·MgO), zirconia (ZrO₂), silica-zirconia (SiO₂·ZrO₂), ZnO·ZrO₂, and Al₂O₃·B₂O₃.

8. The process for producing a carbonate compound according to any one of claims 1 to 7, wherein the fluorine-containing compound having a carbonate bond is a compound represented by the following formula (31) or a compound represented by the following formula (32): 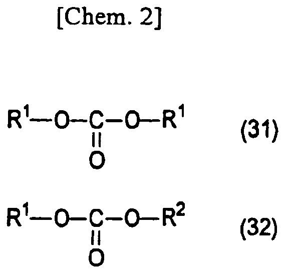 wherein R¹ and R² each represents a monovalent fluorine-containing aliphatic hydrocarbon group or a monovalent fluorine-containing aromatic hydrocarbon group, provided that R¹ and R² are not the same group.

9. The process for producing a carbonate compound according to any one of claims 1 to 7, wherein the fluorine-containing compound having a carbonate bond is a cyclic carbonate compound represented by the following formula (3a): 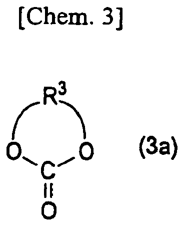 wherein R³ represents a divalent fluorine-containing aliphatic hydrocarbon group or a divalent fluorine-containing aromatic hydrocarbon group.

10. The process for producing a carbonate compound according to any one of claims 1 to 7, wherein the fluorine-containing compound having a carbonate bond is a linear carbonate compound represented by the following formula (3b): 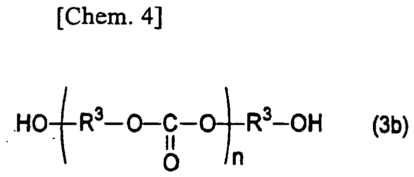 wherein R³ represents a divalent fluorine-containing aliphatic hydrocarbon group or a divalent fluorine-containing aromatic hydrocarbon group.

11. The process for producing a carbonate compound according to any one of claims 1 to 8, wherein the fluorine-containing compound having at least one OH group comprises at least one member selected from the group consisting of 2,2,2-trifluoroethanol, 2,2,3,3,3-pentafluoropropanol, 2,2,3,3-tetrafluoropropanol, 1-trifluoromethyl-2,2,2-trifluoro-1-ethanol (hexafluoroisopropanol), 2,2,3,4,4,4-hexafluorobutanol, 2,2,3,3,4,4,5,5-octafluoropentanol, 2,2-difluoro-2-(1,1,2,2-tetrafluoro-2-(pentafluoroethoxy)ethoxy)ethanol (CF₃CF₂OCF₂CF₂OCF₂CH₂OH), 2,2-difluoro-2-(tetrafluoro-2-(tetrafluoro-2-(pentafluoroethoxy)ethoxy)ethoxy)ethanol (CF₃CF₂OCF₂CF₂OCF₂CF₂OCF₂CH₂OH), 2,3,3,3-tetrafluoro-2-(1,1,2,3,3,3-hexafluoro-2-(1,1,2,2,3,3,3-heptafluoropropoxy)propoxy)-1-propanol (CF₃CF₂CF₂OCF(CF₃)CF₂OCF(CF₃)CH₂OH), 2,2,3,3-tetrafluoro-2-(1,1,2,2,3,3,3-heptafluoropropoxy)-1-propanol (CF₃CF₂CF₂OCF(CF₃)CH₂OH), and fluorine-containing phenols.

12. The process for producing a carbonate compound according to any one of claims 1 to 7, 9, and 10, wherein the fluorine-containing compound having at least one OH group comprises at least one member selected from the group consisting of 3,3,3-trifluoro-1,2-propanediol, 4,4,4,3,3-pentafluoro-1,2-butanediol, 1,1,1,4,4,4-hexafluoro-2,3-butanediol,3,3,4,4-tetrafluoro-1,6-hexanediol, 3,3,4,4,5,5,6,6-octafluoro-1,8-octanediol, tetrafluorohydroquinone, tetrafluororesorcinol, 2,2-bis(4-hydroxyphenyl)-hexafluoropropane, and a compound represented by the following formula (X):
HO-CH₂CF₂-(CF₂CF₂O)ₘ-CF₂CH₂-OH (X)
wherein m is an integer of 2 to 30.

13. The process for producing a carbonate compound according to any one of claims 1 to 12, wherein the reaction is carried out with removing formed CHX¹X²X³ and/or CHX⁴X⁵X⁶ from the reaction system by distillation.

## Patentansprüche

1. Verfahren zur Herstellung einer Carbonat-Verbindung, umfassend das Umsetzen einer Verbindung, dargestellt durch die nachstehende Formel (1), mit einer Fluor-enthaltenden Verbindung mit mindestens einer OH-Gruppe in Gegenwart eines Katalysators zum Erhalt einer Fluor-enthaltenden Verbindung mit einer Carbonatbindung: 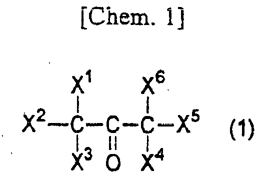 wobei X¹ bis X⁶ jeweils ein Chloratom darstellen, und wobei die Fluorenthaltende Verbindung mit mindestens einer OH-Gruppe ein Polyfluoralkanmonool mit 2 bis 10 Kohlenstoffatomen, das kein Fluoratom in der α-Stellung aufweist und ein etherisches Sauerstoffatom aufweisen kann, oder ein Polyfluoralkandiol mit 3 bis 10 Kohlenstoffatomen, das kein Fluoratom in der α-Stellung aufweist und ein etherisches Sauerstoffatom aufweisen kann, ist.

2. Verfahren zur Herstellung einer Carbonatverbindung nach Anspruch 1, wobei der Katalysator ein Halogensalz umfaßt.

3. Verfahren zur Herstellung einer Carbonatverbindung nach Anspruch 2, wobei das Halogensalz ein oder mehrere, ausgewählt aus der Gruppe, bestehend aus Halogensalzen von Alkalimetallen, Halogensalzen von Erdalkalimetallen, Ammoniumhalogensalzen, quartäre Ammoniumhalogensalze und Ionenaustauscherharze mit einer Halogensalzstruktur, umfaßt.

4. Verfahren zur Herstellung einer Carbonatverbindung nach Anspruch 2 oder 3, wobei das Halogensalz ein Fluorid eines Alkalimetalls ist.

5. Verfahren zur Herstellung einer Carbonatverbindung nach einem der Ansprüche 1 bis 4, wobei die Umsetzung in Gegenwart des Katalyators und eines Promotors durchgeführt wird, wobei der Promotor ein fester Säurekatalysator ist.

6. Verfahren zur Herstellung einer Carbonatverbindung nach Anspruch 5, wobei der feste Säurekatalysator mindestens einen, ausgewählt aus der Gruppe, bestehend aus Metalloxiden mit einer hohen Säurestärke, Heteropolysäuren und Kationenaustauscherharzen, umfaßt.

7. Verfahren zur Herstellung einer Carbonatverbindung nach Anspruch 6, wobei die Metalloxide mit einer hohen Säurestärke mindestens eines, ausgewählt aus der Gruppe, bestehend aus Ceriumoxid (CeO₂/Ce₂O₃), Siliciumoxid-Aluminumoxid (SiO₂·Al₂O₃), γ-Aluminiumoxid (Al₂O₃), Siliciumoxid-Magnesiumoxid (SiO₂·MgO), Zirconiumoxid (ZrO₂), Siliciumoxid-Zirconiumoxid (SiO₂·ZrO₂), ZnO·ZrO₂ und Al₂O₃·S₂O₃, umfaßt.

8. Verfahren zur Herstellung einer Carbonatverbindung nach einem der Ansprüche 1 bis 7, wobei die Fluor-enthaltende Verbindung mit einer Carbonatbindung eine Verbindung, dargestellt durch die nachstehende Formel (31), oder eine Verbindung, dargestellt durch die nachstehende Formel (32) ist: 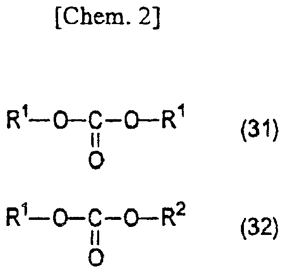 wobei R¹ und R² jeweils eine einwertige Fluor-enthaltende aliphatische Kohlenwasserstoffgruppe oder eine einwertige Fluor-enthaltende aromatische Kohlenwasserstoffgruppe darstellt, mit der Maßgabe, daß R¹ und R² nicht die gleiche Gruppe ist.

9. Verfahren zur Herstellung einer Carbonatverbindung nach einem der Ansprüche 1 bis 7, wobei die Fluor-enthaltende Verbindung mit einer Carbonatbindung eine cyclische Carbonatverbindung, dargestellt durch die nachstehende Formel (3a), ist: 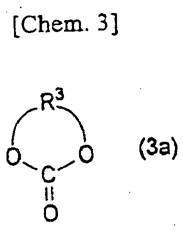 wobei R³ eine zweiwertige Fluor-enthaltende aliphatische Kohlenwasserstoffgruppe oder eine zweiwertige Fluor-enthaltende aromatische Kohlenwasserstoffgruppe darstellt.

10. Verfahren zur Herstellung einer Carbonatverbindung nach einem der Ansprüche 1 bis 7, wobei die Fluor-enthaltende Verbindung mit einer Carbonatbindung eine lineare Carbonatverbindung, dargestellt durch die nachstehende Formel (3b), ist: 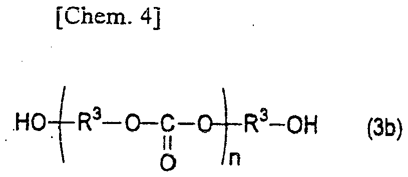 wobei R³ eine zweiwertige Fluor-enthaltende aliphatische Kohlenwasserstoffgruppe oder eine zweiwertige Fluor-enthaltende aromatische Kohlenwasserstoffgruppe darstellt.

11. Verfahren zur Herstellung einer Carbonatverbindung nach einem der Ansprüche 1 bis 8, wobei die Fluor-enthaltende Verbindung mit mindestens einer OH-Gruppe mindestens eine, ausgewählt aus der Gruppe, bestehend aus 2,2,2-Trifluorethanol, 2,2,3,3,3-Pentafluorpropanol, 2,2,3,3-Tetrafluorpropanol, 1-Trifluormethyl-2,2,2-trifluor-1-ethanol(hexafluorisopropanol), 2,2,3,4,4,4-Hexafluorbutanol, 2,2,3,3,4,4,5,5-Octafluorpentanol, 2,2-Difluor-2-(1,1,2,2-tetrafluor-2-(pentafluorethoxy)ethoxy)ethanol (CF₃CF₂OCF₂CF₂OCF₂CH₂OH), 2,2-Difluor-2-(tetrafluor-2-(tetrafluor-2-(pentafluorethoxy)ethoxy)ethoxy)ethanol (CF₃CF₂OCF₂CF₂OCF₂CF2OCF2CH₂OH), 2,3,3,3-Tetrafluor-2-(1,1,2,3,3,3-hexafluor-2-(1,1,2,2,3,3,3-heptafluorpropoxy)propoxy)-1-propanol (CF₃CF₂CF₂OCF(CF₃)CF₂OCF(CF₃)CH₂OH), 2,2,3,3-Tetrafluor-2-(1,1,2,2,3,3,3-heptafluorpropoxy)-1-propanol(CF₃CF₂CF₂OCF(CF₃)CH₂OH) und Fluor-enthaltende Phenole, umfaßt.

12. Verfahren zur Herstellung einer Carbonatverbindung nach einem der Ansprüche 1 bis 7, 9 und 10, wobei die Fluor-enthaltende Verbindung mit mindestens einer OH-Gruppe mindestens eine, ausgewählt aus der Gruppe, bestehend aus 3,3,3-Trifluor-1,2-propandiol, 4,4,4,3,3-Pentafluor-1,2-butandiol, 1,1,1,4,4,4-Hexafluor-2,3-butandiol, 3,3,4,4-Tetrafluor-1,6-hexandiol, 3,3,4,4,5,5,6,6-Octafluor-1,8-octandiol, Tetrafluorhydrochinon, Tetrafluorresorcinol, 2,2-Bis(4-hydroxyphenyl)-hexafluorpropan und eine Verbindung, dargestellt durch die nachstehende Formel (X), umfaßt:
HO-CH₂CF₂-(CF₂CF₂O)ₘ-CF₂CH₂-OH (X)
wobei m eine ganze Zahl von 2 bis 30 ist.

13. Verfahren zur Herstellung einer Carbonatverbindung nach einem der Ansprüche 1 bis 12, wobei die Umsetzung unter Entfernen von gebildetem CHX¹X²X³ und/oder CHX⁴X⁵X⁶ aus dem Reaktionssystem durch Abdampfung ausgeführt wird.

## Revendications

1. Procédé pour produire un composé carbonate, comprenant la réaction d'un composé représenté par la formule (1) suivante avec un composé fluoré ayant au moins un groupe OH, en présence d'un catalyseur, pour obtenir un composé fluoré ayant une liaison carbonate : 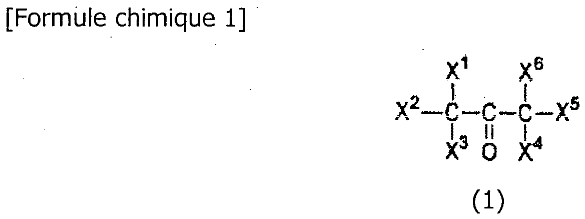 dans laquelle chacun de X¹ à X⁶ représente un atome de chlore,
dans lequel le composé fluoré ayant au moins un groupe OH est un polyfluoroalcanemonool ayant 2 à 10 atomes de carbone et n'ayant pas d'atome de fluor en position α et pouvant avoir un atome d'oxygène d'éther, ou un polyfluoroalcanediol ayant 3 à 10 atomes de carbone et n'ayant pas d'atome de fluor en position α et pouvant avoir un atome d'oxygène d'éther.

2. Procédé pour produire un composé carbonate selon la revendication 1, dans lequel le catalyseur comprend un sel halogéné.

3. Procédé pour produire un composé carbonate selon la revendication 2, dans lequel le sel halogéné comprend un ou plusieurs membres choisis dans l'ensemble constitué par les sels halogénés de métaux alcalins, les sels halogénés de métaux alcalino-terreux, les sels halogénés d'ammonium, les sels halogénés d'ammonium quaternaire, et les résines échangeuses d'ions ayant une structure de sel halogéné.

4. Procédé pour produire un composé carbonate selon la revendication 2 ou 3, dans lequel le sel halogéné est un fluorure d'un métal alcalin.

5. Procédé pour produire un composé carbonate selon l'une quelconque des revendications 1 à 4, dans lequel la réaction se déroule en présence du catalyseur et d'un promoteur, et dans lequel le promoteur est un catalyseur acide solide.

6. Procédé pour produire un composé carbonate selon la revendication 5, dans lequel le catalyseur acide solide comprend au moins un membre choisi dans l'ensemble constitué par les oxydes métalliques ayant un fort indice d'acide, les hétéropolyacides, et les résines échangeuses de cations.

7. Procédé pour produire un composé carbonate selon la revendication 6, dans lequel les oxydes métalliques ayant un fort indice d'acide comprennent au moins un membre choisi dans l'ensemble constitué par l'oxyde de cérium (CeO₂/Ce₂O₃), la silice-alumine (SiO₂•Al₂O₃), la γ-alumine (Al₂O₃), la silice-magnésie (SiO₂•MgO), la zircone (ZrO₂), la silice-zircone (SiO₂•ZrO₂), ZnO•ZrO₂, et Al₂O₃•B₂O₃.

8. Procédé pour produire un composé carbonate selon l'une quelconque des revendications 1 à 7, dans lequel le composé fluoré ayant une liaison carbonate est un composé représenté par la formule (31) suivante ou un composé représenté par la formule (32) suivante : 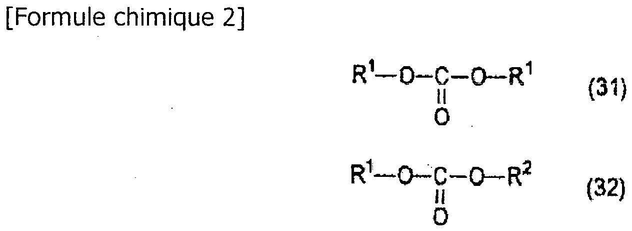 où chacun de R¹ et R² représente un groupe hydrocarboné aliphatique fluoré monovalent ou un groupe hydrocarboné aromatique fluoré monovalent, sous réserve que R¹ et R² ne représentent pas le même groupe.

9. Procédé pour produire un composé carbonate selon l'une quelconque des revendications 1 à 7, dans lequel le composé fluoré ayant une liaison carbonate est un composé carbonate cyclique représenté par la formule (3a) suivante : 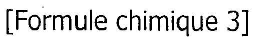 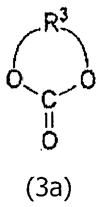 dans laquelle R³ représente un groupe hydrocarboné aliphatique fluoré divalent ou un groupe hydrocarboné aromatique fluoré divalent.

10. Procédé pour produire un composé carbonate selon l'une quelconque des revendications 1 à 7, dans lequel le composé fluoré ayant une liaison carbonate est un composé carbonate linéaire représenté par la formule (3b) suivante : 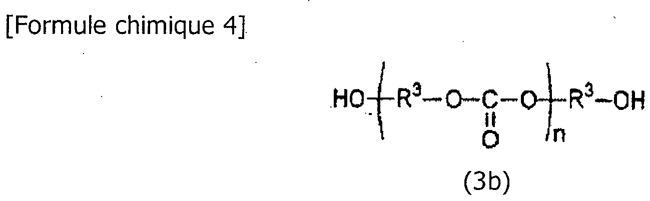 dans laquelle R³ représente un groupe hydrocarboné aliphatique fluoré divalent ou un groupe hydrocarboné aromatique fluoré divalent.

11. Procédé pour produire un composé carbonate selon l'une quelconque des revendications 1 à 8, dans lequel le composé fluoré ayant au moins un groupe OH comprend au moins un membre choisi dans l'ensemble constitué par le 2,2,2-trifluoroéthanol, le 2,2,3,3,3-pentafluoropropanol, le 2,2,3,3-tétrafluoropropanol, le 1-trifluorométhyl-2,2,2-trifluoro-1-éthanol (hexafluoroisopropanol), le 2,2,3,4,4,4-hexafluorobutanol, le 2,2,3,3,4,4,5,5-octafluoropentanol, le 2,2-difluoro-2-(1,1,2,2-tétrafluoro-2-(pentafluoroéthoxy)éthoxy)éthanol (CF₃CF₂OCF₂CF₂OCF₂CH₂OH), le 2,2-difluoro-2-(tétrafluoro-2-(tétrafluoro-2-(pentafluoroéthoxy)éthoxy)éthoxy)éthanol (CF₃CF₂OCF₂CF₂OCF₂CF₂OCF₂CH₂OH), le 2,3,3,3-tétrafluoro-2-(1,1,2,3,3,3-hexafluoro-2-(1,1,2,2,3,3,3-heptafluoropropoxy)propoxy)-1-propanol (CF₃CF₂CF₂OCF(CF₃)CF₂OCF(CF₃)CH₂OH), le 2,2,3,3-tétrafluoro-2-(1,1,2,2,3,3,3-heptafluoropropoxy)-1-propanol (CF₃CF₂CF₂OCF(CF₃)CH₂OH) et les phénols fluorés.

12. Procédé pour produire un composé carbonate selon l'une quelconque des revendications 7, 9 et 10, dans lequel le composé fluoré ayant au moins un groupe OH comprend au moins un membre choisi dans l'ensemble constitué par le 3,3,3-trifluoro-1,2-propanediol, le 4,4,4,3,3-pentafluoro-1,2-butanediol, le 1,1,1,4,4,4-hexafluoro-2,3-butanediol, le 3,3,4,4-tétrafluoro-l,6-hexanediol, le 3,3,4,4,5,5,6,6-octafluoro-1,8-octanediol, la tétrafluorohydroquinone, le tétrafluororésorcinol, le 2,2-bis(4-hydroxyphényl)hexafluoropropane, et un composé représenté par la formule (X) suivante :
HO-CH₂CF₂-(CF₂CF₂O)ₘ-CF₂CH₂-OH (X)
dans laquelle m est un entier de 2 à 30.

13. Procédé pour produire un composé carbonate selon l'une quelconque des revendications 1 à 12, dans lequel la réaction se déroule avec élimination, par distillation du système réactionnel, des CHX¹X²X³ et/ou CHX⁴X⁵X⁶ formés.
